# EUROPÄISCHE PATENTANMELDUNG

(11) **EP 4 062 911 A1**
(43) Veröffentlichungstag der Anmeldung: **28.09.2022**
(21) Anmeldenummer: 21165271.4
(22) Anmeldetag: 26.03.2021
(51) Int. Cl.: A61K 31/426, A61K 31/427, A61K 31/4439, A61K 31/517, A61K 45/06, A61P 9/00

(54) **VERWENDUNG VON PPARY-AGONISTEN ZUR BEHANDLUNG VON PRIMÄREN, ANGEBORENEN ERKRANKUNGEN DES HERZMUSKELS (KARDIOMYOPATHIEN)**

(71) Anmelder: Preventage Therapeutics GmbH, 82319 Starnberg (DE)
(72) Erfinder: Dr. Wolf, Cordula, 82319 Starnberg (DE)
(74) Vertreter: Graf von Stosch Patentanwaltsgesellschaft mbH

(57) **Zusammenfassung**

Die vorliegende Erfindung betrifft PPARy-Agonisten, insbesondere aus der Gruppe der Thiazolidin-2,4-dione, zur Verwendung in einem Verfahren zur Prävention oder Therapie einer primären, angeborenen Kardiomyopathie.

## Beschreibung

Als primäre, angeborene Kardiomyopathien werden alle Erkrankungen des Herzmuskels bezeichnet, die genetisch verursacht sind. Sie treten zunächst entweder isoliert als Herzmuskelerkrankungen in Erscheinung (wie zum Beispiel die hypertrophe Kardiomyopathie, die dilatative Kardiomyopathie, die arrhythmogene rechtsventrikuläre Kardiomyopathie, oder die restriktive Kardiomyopathie), oder sie treten als Herzmuskelerkrankungen im Rahmen eines angeborenen strukturellen Herzfehlers, eines genetisch verursachten Syndroms (wie zum Beispiel im Rahmen einer Erkrankung aus dem Noonan-Syndrom Spektrum oder anderer RASopathien), einer angeborenen Stoffwechselerkrankung (wie zum Beispiel im Rahmen des Morbus Fabry, der Mitochondriopathie, oder von Glykogenspeichererkrankungen) oder einer angeborenen neuromuskulären Erkrankung (wie zum Beispiel im Rahmen der Duchenne Muskeldystrophie oder der myotonen Dystrophie) auf¹.

Primäre, angeborene Kardiomyopathien entstehen also nicht sekundär im Rahmen anderer Erkrankungen (wie zum Beispiel im Rahmen eines arteriellen Hypertonus, eines Diabetes mellitus, eines metabolischen Syndroms oder einer koronaren Herzerkrankung) und grenzen sich somit als eigenständige Krankheitsbilder von den sekundären myokardialen Hypertrophien ab. Der Ausschluss solcher sekundärer Erkrankungen ist ein wesentlicher Bestandteil der klinischen Diagnose der primären angeborenen, insbesondere der hypertrophen Kardiomyopathie (^{2, 3, 4, 5, 1}).

Das klinische Erscheinungsbild primärer, angeborener Kardiomyopathien ist insbesondere durch eine Verdickung (Hypertrophie) des Herzmuskelgewebes (Myokards), durch eine Vergrößerung (Dilatation) des Herzens oder durch eine Kombination von beiden Veränderungen gekennzeichnet. Auch kann es bei primären, angeborenen Kardiomyopathien zu einer begleitenden Vernarbung (Fibrosierung) des Herzmuskels kommen. Im Verlauf der Erkrankung tritt schließlich eine Reduktion der Füllfunktion (diastolische Dysfunktion) oder der Auswurffunktion (systolische Dysfunktion) des Herzens als Erscheinungsbild hinzu. Dies geht mit der klinischen Symptomatik einer Herzinsuffizienz einher. Häufig werden primäre, angeborene Kardiomyopathien von Herzrhythmusstörungen begleitet, die mit der klinischen Symptomatik von z.B. Tachykardien und Synkopen bis hin zum plötzlichen Herztod assoziiert sind. Die Variabilität des klinischen Erscheinungsbilds erstreckt sich also über einen weiten Bereich - sowohl hinsichtlich der klinischen Symptomatik, des Schweregrads der Symptome als auch des Zeitpunkts der klinischen Manifestation. Bereits Neugeborene können von einem schweren Verlauf betroffen sein; die Erkrankung kann sich aber auch erst im frühen oder späteren Erwachsenenalter manifestieren.

Als eigenständiges Krankheitsbild innerhalb der angeborenen, primären Kardiomyopathien wird die hypertrophe Kardiomyopathie (HCM) definiert. Die HCM ist bei weitem die häufigste primäre, angeborene Kardiomyopathie. Im Allgemeinen stellt die HCM mit einer Prävalenz von etwa 1:250 bis 1:500 auch die häufigste vererbte, genetisch bedingte Herzerkrankung dar ¹. Die hypertrophe Kardiomyopathie unterscheidet sich pathogenetisch und pathophysiologisch von einer Herzmuskelverdickung, welche, wie zuvor beschreiben, lediglich sekundär im Rahmen anderer Erkrankungen, zum Beispiel des arteriellen Hypertonus, von Herzklappenvitien, des Diabetes mellitus, oder von Stoffwechsel-, neurologischen oder syndromalen Erkrankungen zu beobachten ist. Daher erfordert die klinische Diagnose der HCM unter anderem den Ausschluss solcher sekundärer Erkrankungen (^{2, 3, 4, 5, 1}) und wird nach den Richtlinien der US-amerikanischen und europäischen Fachgesellschaften wie folgt definiert:
"... For the purposes of this guideline, we have considered the clinical definition of HCM as a disease state in which morphologic expression is confined solely to the heart. It is characterized predominantly by LVH in the absence of another cardiac, systemic, or metabolic disease capable of producing the magnitude of hypertrophy evident in a given patient and for which a diseasecausing sarcomere (or sarcomere-related) variant is identified, or genetic etiology remains unresolved." ⁶
"... The generally accepted definition of HCM is a disease state characterized by unexplained LV hypertrophy associated with nondilated ventricular chambers in the absence of another cardiac or systemic disease that itself would be capable of producing the magnitude of hypertrophy evident in a given patient
..." (2011 ACCF/AHA Guideline for the Diagnosis and Treatment of Hypertrophic Cardiomyopathy: Executive Summary ⁵)
"... Left ventricular wall thickening is associated with a nondilated and hyperdynamic chamber (often with systolic cavity obliteration) in the absence of another cardiac or systemic disease ..." (American College of Cardiology / European Society of Cardiology Clinical Expert Consensus Document on Hypertrophic Cardiomyopathy ³)

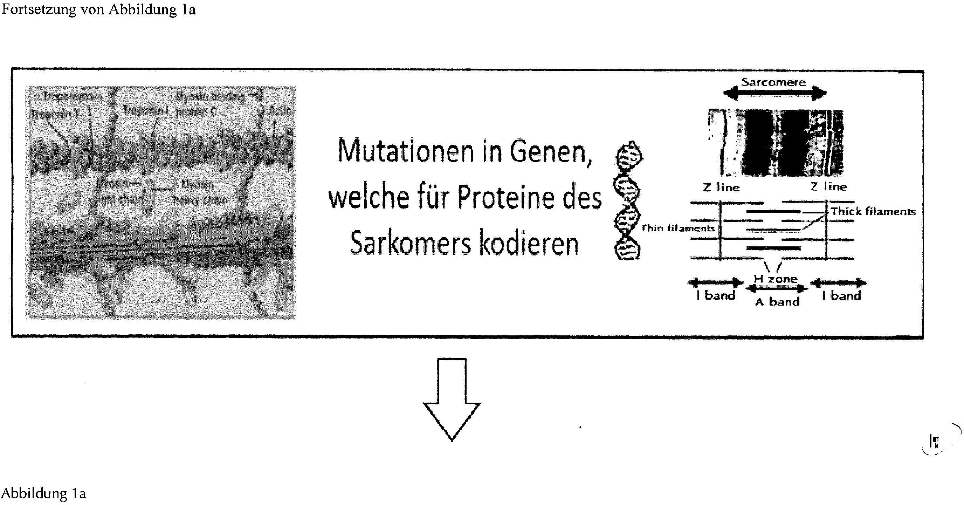

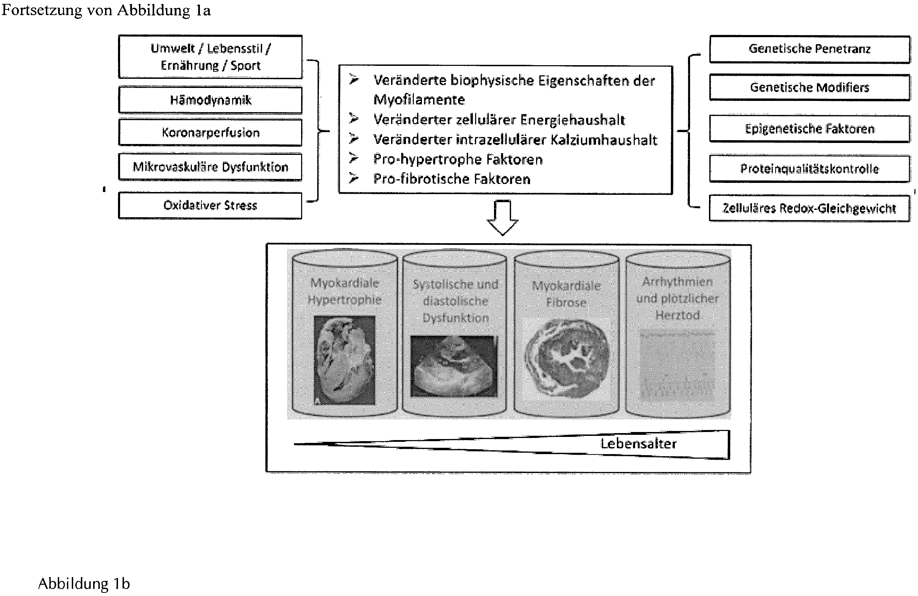

Wie bereits zuvor für primäre, angeborene Kardiomyopathien im allgemeinen dargelegt, erweist sich auch bei der HCM mit zunehmendem Lebensalter die Herzmuskelverdickung (Hypertrophie) und die Vernarbung (Fibrose) des Myokards als progredient ². Dies führt klinisch zu kardialer Dysfunktion, zu Herzinsuffizienz und zu Herzrhythmusstörungen, welche das Risiko für den plötzlichen Herztod erhöhen. Als genetische Ursache der HCM sind zumeist Mutationen in Genen, die für Proteine des Sarkomers kodieren, zu ermitteln ^{3, 4}. Die entsprechend modifizierten strukturellen Proteine der Herzmuskelzelle verändern deren biophysische Kontraktilität ⁵, den zellulären Energie- ⁶ und Kalzium-Haushalt ⁷, und induzieren pro-hypertrophe und pro-fibrotische Signalwege ^{8, 9, 10}. Zusammenfassend sind diese Zusammenhänge in Figur 1 dargestellt.

Allerdings lässt sich nicht grundsätzlich bei Verdacht auf HCM mit genetischen Standardtests eine Veränderung im Genom des Patienten eindeutig nachweisen. Möglicherweise sind hierfür unter anderem epigenetische Faktoren verantwortlich oder auch die mangelnde Verfügbarkeit von Herzmuskelgewebe zur Durchführung von genetischen Tests. Denn typischerweise werden genetische Tests mit Hilfe anderer, leichter zu gewinnender Gewebe, wie z.B. Blut- oder Epithelzellen, durchgeführt, so dass in diesen ein etwaiges genetisches Mosaik mit den charakteristischen Mutationen des Herzmuskelgewebes nicht erkannt werden kann. Der Verlauf der Erkrankung variiert stark, da neben der genetischen Prädisposition multiple Faktoren den Schweregrad der Erkrankung beeinflussen ^{11, 12, 13, 14, 15} (Figur 1).

Pharmakologische Ansätze zur Therapie der primären, angeborenen Kardiomyopathien im allgemeinen und der hypertrophen Kardiomyopathie im besonderen, sind im Stand der Technik, wenn überhaupt, allenfalls Gegenstand präklinischer oder klinischer Untersuchungen, haben aber noch keine Anwendung in der klinischen Praxis erfahren. In Tiermodellen wurde vorläufig ein positiver Effekt des Kalzium-Antagonisten Diltiazem ^{7, 8} und des Angiotensin II-Rezeptor Antagonisten Losartan ⁹ beschrieben. Erste klinische Studien zeigen, dass die Einnahme von Diltiazem das linksventrikuläre Remodeling verlangsamen könnte ¹⁰. Die klinischen Ergebnisse beim Einsatz von Losartan im Stand der Technik sind widersprüchlich ^{11, 12}. Weiterhin wurde im Stand der Technik die Inhibition der kardialen Myosin-ATPase durch MYK-461 ¹³ im Mausmodell untersucht. Vor kurzem wurde ein positiver Effekt dieses kardialen Myosin-Inhibitors (Mavacamten) bei Patienten mit obstruktiver Kardiomyopathie in einer klinischen Phase III Studie gezeigt ¹⁴.

Aufgrund der mangelnden Verfügbarkeit wirksamer kausaler oder krankheitsmodifizierender pharmakologischer Therapieansätze wird die hypertrophe Kardiomyopathie daher nach erfolgter Diagnose zumeist ausschließlich konservativ behandelt, z.B. durch Vermeidung allzu starker Belastung des Herzens bzw. Verzicht auf Leistungssport, und in regelmäßigen Abständen kontrolliert. Der Fokus der ärztlichen Intervention liegt auf der Prävention des plötzlichen Herztodes, auf der symptomatischen medikamentösen Behandlung einer gegebenenfalls auftretenden Herzinsuffizienz, z.B. mit Betablockern, ACE-Hemmern und Kalzium-Antagonisten, sowie auf dem Versuch einer Limitierung des Fortschreitens des myokardialen Umbaus mittels konservativer Maßnahmen ^{16, 17}. In schweren Fällen wird ggf. die Implantierung eines Kardioverter-Defibrillators (ICD) erwogen, der den Patienten ggf. vor frühzeitigem Tod durch akuten Herzstillstand bewahren, aber das Fortschreiten der Erkrankung nicht aufhalten kann. Bei Vorliegen einer hochgradigen Verengung des linksventrikulären Ausflusstraktes durch den verdickten Herzmuskel kann eine Operation am offenen Herzen (operative septale Myektomie) oder eine transkoronare Ablation der Septumhypertrophie (TASH) erforderlich sein. Eine Herztransplantation stellt wegen mangelnder Verfügbarkeit von Spenderorganen, wegen der Invasivität der Operation und der damit verbundenen Risiken sowie wegen der danach notwendigen lebenslangen Immunsuppression nur für sehr wenige Patienten eine Behandlungsalternative dar.

Aufgabe der vorliegenden Erfindung ist es in Anbetracht all dessen, eine pharmakologische Behandlungsoption zur Prävention und Behandlung von primären, angeborenen Kardiomyopathien, insbesondere zur Prävention und Behandlung der hypertrophen Kardiomyopathie (HCM) zu eröffnen.

Gelöst wird diese Aufgabe durch die überraschende Erkenntnis, daß eine pharmakologische Intervention in Form einer Aktivierung des Peroxisom-Proliferator-aktivierten-Rezeptor gamma (PPARγ) Signalwegs ein hochwirksames präventives oder therapeutisches Potential für das obige Krankheitsspektrum bietet. Daher wird erfindungsgemäß ein PPARγ-Agonist zur Verwendung in einem Verfahren zur Prävention oder Therapie der primären, angeborenen Kardiomyopathien bereitgestellt. Insbesondere können als primäre, angeborene Kardiomyopathien die hypertrophe Kardiomyopathie (HCM), die dilatative Kardiomyopathie (DCM), die restriktive HCM sowie eine Kardiomyopathie bei strukturellen angeborenen Herzfehlern, bei Erkrankungen aus dem Noonan-Syndrom-Spektrum oder anderen RASopathien und bei anderen angeborenen Systemerkrankungen präventiv oder therapeutisch behandelt werden, ganz besonders die hypertrophe Kardiomyopathie (HCM).
Im menschlichen Organismus sind drei PPAR-Subtypen (α, β/δ, γ) bekannt, deren gewebespezifische Expression und insbesondere deren Genexpressionsmuster ebenso unterschiedlich sind wie die biologische Funktion der Gene, deren Transkription beeinflusst wird ^{15, 16}. PPARγ wird ubiquitär exprimiert ^{17, 18}.

Die erfindungsgemäß eingesetzten PPARy-Agonisten oder -Modulatoren greifen demnach in die Signalkaskade am Peroxisom-Proliferator-aktivierten Rezeptor-Signalweg ein. Peroxisom-Proliferator-aktivierte Rezeptoren (PPARs) gehören zu einer Gruppe von Rezeptoren, die im Zellkern angesiedelt sind ¹⁹. Sie können über einen physiologischen oder pharmakologischen Liganden aktiviert werden und als Transkriptionsfaktoren die Expression einer Vielzahl von Genen regulieren. Nach der Aktivierung erfolgt eine Bindung der PPARs an einen ebenfalls aktivierten Retinoid-X-Rezeptor (RXR). Im Anschluss bindet dieser Komplex an eine spezifische DNA-Sequenz, das PPAR Response Element (PPRE), und induziert dadurch spezifische Gentranskriptionsmuster ^{20, 21, 22}.

Erfindungsgemäß wird also ein PPARy-Agonist oder -Modulator als Wirkstoff zur entsprechenden Prävention oder Therapie offenbart, insbesondere ein Wirkstoff aus der Wirkstoffklasse der Thiazolidindione, wie z.B. Pioglitazon oder Rosiglitazon, zur Prävention und Behandlung von primären, angeborenen Kardiomyopathien, insbesondere zur Prävention und Behandlung der HCM.

Der als PPARy-Agonist eingesetzte Wirkstoff oder ein Glitazon kann ein Enantiomer, Tautomer, Hydrat, Solvat oder Salz des entsprechenden Wirkstoffs sein.

Die Wirkstoffklasse der Thiazolidindione ist seit Jahrzehnten aus anderem Zusammenhang bekannt. Ende der 80er Jahre wurden erstmals anti-diabetische Effekte von Thiazolidindionen beschrieben ^{23, 24, 25}. Sie steigern die Insulinsensitivität und verhindern eine Hyperinsulinämie. Die Thiazolidindione werden daher auch als Insulin-Sensitizer ^{26, 27, 28, 29, 30} bezeichnet. So etwa wurde ein Vertreter dieser Wirkstoffklasse, Rosiglitazon, zur Behandlung des Diabetes mellitus Typ 2 (Handelsname: Avandia, GlaxoSmithKline) eingesetzt; aufgrund der Beobachtung kardiovaskulärer Nebenwirkungen, einhergehend mit einer deutlichen Erhöhung des Herzinfarktrisikos, wurde er jedoch in etlichen Ländern inzwischen vom Markt genommen. Insgesamt läßt sich feststellen, daß sich alle im Stand der Technik erhobenen Daten und Untersuchungen, soweit sie sich mit dem PPARγ-Signalweg im allgemeinen und mit dem therapeutischen Potential von PPARy-Agonisten, wie der Thiazolidindione, im besonderen befassen, auf kardiale Erkrankungen beziehen, die sekundär als Folge anderer zugrundeliegender Erkrankungen entstehen, wie zum Beispiel des Diabetes mellitus, der arteriellen Hypertension, des metabolischen Syndroms oder der pulmonalen Hypertension ^{31, 32}.

Im Zuge der experimentellen präklinischen Untersuchungen zur vorliegenden Erfindung hat sich - insoweit umso überraschender - erwiesen, dass PPARγ-Agonisten, insbesondere Thiazolidindione, bei der primären, angeborenen, genetisch verursachten Erkrankung "hypertrophe Kardiomyopathie" einen ausgeprägt protektiven Effekt auf das Herzmuskelgewebe und die Herzfunktion ausüben können. Diese Beobachtung steht der vorherrschenden Lehrmeinung und gängigen medizinischen Praxis diametral entgegen. Ohne eine abschließende Erklärung für diese abweichenden Beobachtungen bieten zu können, könnte deren Grundlage eine deutlich abweichende Pathogenese der primären, genetisch bedingten Kardiomyopathien gegenüber sekundären Herzmuskelerkrankungen sein. Während die erfindungsgemäß adressierten Erkrankungen als Folge angeborener Veränderungen im Erbgut zu verstehen sind, welche die entsprechenden Herzmuskelveränderungen von Geburt an verursachen und zu einer progredienten Entwicklung führen, liegen den sekundären Herzmuskelerkrankungen grundsätzlich andere Ätiologien zugrunde.

Erfindungsgemäß ist der zur Prävention oder zur Therapie eingesetzte PPARγ-Agonist in einer Ausführungsform eine Verbindung aus der Gruppe der Thiazolidin-2,4-dione. Die Ringstruktur des Thiazolidin-2,4-dions ist das charakteristische Grundgerüst der Verbindungen dieser Verbindungsklasse, wobei typischerweise ein vergleichsweiser langkettiger Substituent am C5-Ringatom eingeführt ist. Vorzugsweise handelt es sich also um eine C5-substituiertes Thiazolidin-2,4-dion. Der Substituent weist vorzugsweise zwei über eine Linkerstruktur miteinander verbundene (hetero)aromatische Ringsysteme auf. Die Ringsysteme können bspw. aromatische 6-Ringsysteme, insbesondere ein Pyridylring, ein Naphthylring oder ein Benzylring, sein. In einer Ausführungsform wird ein Glitazon, insbesondere eines oder mehrere der folgenden Glitazone ausgewählt aus der Gruppe, bestehend aus Ciglitazon, Balaglitazon, Darglitazon, Englitazon, Netoglitazon, Pioglitazon, Rivoglitazon, Rosiglitazon, GQ-16, und Troglitazon, bereitgestellt und erfindungsgemäß eingesetzt. In einer spezifischen Ausführungsform wird erfindungsgemäß Pioglitazon ((*RS*)-5-{*p*-[2-(5-Ethyl-2-pyridyl)ethoxy]benzyl}-2,4-thiazolidindion) oder Rosiglitazon ((*RS*)-5-({4-[2-(Methyl-2-pyridinylamino)ethoxy]phenyl}methyl)-2,4-thiazolidindion) zur Prävention oder Behandlung der vorbeschriebenen Erkrankungen eingesetzt.

Im Falle einer erfindungsgemäßen Verwendung von Vertretern der Wirkstoffklasse der Glitazone ist neben deren PPARy-agonistischer Wirkung auch auf deren Eigenschaft als Inhibitoren des mitochondrialen Pyruvat Carriers (MPC) ³⁴ hinzuweisen. Ohne an eine Theorie gebunden zu sein, kann der Wirkmechanismus der Glitazone damit zusätzlich oder alternativ auch auf deren Eigenschaft als MPC-Inhibitor beruhen. Insoweit werden erfindungsgemäß eingesetzte Glitazone vorliegend auch als MPC-Inhibitoren offenbart. Damit offenbart die vorliegende Erfindung auch MPC-Inhibitoren, insbesondere eine Verbindung aus der Gruppe der Glitazone, zur Verwendung in einem Verfahren zur Prävention oder Therapie von primären, angeborenen Kardiomyopathien. Alle anspruchsgemäß für PPARγ-Agonisten ausgewiesenen Merkmale werden somit auch erfindungsgemäß auch für MPC-Inhibitoren, insbesondere aus der Gruppe der Glitazone, und deren Verwendung für das vorbezeichnete prophylaktische oder therapeutische Verfahren offenbart.

Auf Grund der Chiralität am C5-Ringatom treten die Glitazone als Enantiomere auf. Einerseits wird erfindungsgemäß ein Racemat der Enantiomere zur Prävention oder Behandlung eingesetzt.

Alternativ kann aber auch eines der beiden Enantiomere in angereicherter, vorzugsweise in Reinform, eingesetzt werden. In Betracht kommen hierbei grundsätzlich sowohl eine angereicherte/Reinform des (*S*)- als auch des (*R*)-Enantiomers. Das (*S*)-Enantiomer kann dann bevorzugt sein, wenn eine erhöhte PPARy-agonistische Wirkung sichergestellt werden soll. Eine Aktivierung des Peroxisom-Proliferator-aktivierten-Rezeptor gamma (PPARγ) wird nämlich fast ausschließlich durch das (*S*)-Enantiomer bewirkt.

Wenn Glitazone in ihrer Eigenschaft als MPC-Inhibitoren erfindungsgemäß eingesetzt werden, können zwar sowohl (*S*)-Enantiomere als auch (*R*)-Enantiomere der Thiazolidindione verabreicht werden. Erfindungsgemäß bevorzugt ist solchenfalls typischerweise jedoch reines bzw. angereichertes (*R*)-Enantiomer. Denn das (*R*)-Enantiomer aktiviert nicht oder nur geringfügig PPARγ, so daß die biologische Wirkung ganz überwiegend durch den MPC-Wirkmechanismus erreicht wird. Auch kann das mit einer insbesondere vom (*S*)-Enantiomer hervorgerufenen PPARy-agonistischen Wirkung verbundene Nebenwirkungsspektrum, bspw. Gewichtszunahme, Ödeme, oder ein erhöhtes Risiko für Frakturen, durch Gabe des (*R*)-Enantiomers als MPC-Inhibitor im wesentlichen unterbunden werden.

Erfindungsgemäß kann weiterhin eine Substitution des Wasserstoffatoms am chiralen Zentrum der Thiazolidindione durch Deuterium insbesondere dann vorgesehen sein, wenn angereicherte oder Reinformen der Enantiomere erfindungsgemäß eingesetzt werden sollen. Hierdurch kann die Kinetik der Racemisierungreaktion nach Gabe von angereicherten oder Reinformen der beiden Enantiomere verändert werden. Denn Thiazolidindione in angereicherter oder reiner Enantiomerform sind in wässriger Lösung in vivo kinetisch nicht stabil und werden innerhalb von einigen Stunden racemisiert. Bei physiologischem pH-Wert von 7,4 und einer Temperatur von 37 °C liegt, ausgehend vom ursprünglich reinen Enantiomer, nach 10 Stunden ein Racemat im Konzentrationsverhältnis von ca. 2:1 (ursprünglich reines Enantiomer : anderes Enantiomer) und nach ca. 48 Stunden im Verhältnis von annähernd 1:1 vor ³⁵. Durch eine Deuterierung am chiralen Zentrum kann sowohl durch das (*S*) als auch durch das (*R*)-Enantiomer ein relativ stärkerer biologischer Effekt, bspw. als PPARγ-Agonist ((*S*)-Enantiomer), bewirkt werden ³³. Im Falle einer bevorzugten Aktivität des Glitazons als MCP-Inhibitor, wird eine deuterierte Form des reinen bzw. angereicherten (*R*)-Enantiomers bevorzugt sein, um die inhibitorische Wirkung auf den mitochondrialen Pyruvat Carrier zu erhöhen. Denn durch die kinetisch verlangsamte Racemisierungsreaktion eines erfindungsgemäß als MPC-Inhibitor einzusetzenden deuterierten (*R*)-Enantiomers wird nur eine geringe oder keine Aktivierung des PPARγ ausgelöst.

PPARy-Agonisten oder MPC-Inhibitoren, insbesondere Glitazone, können bevorzugt als Salze eingesetzt werden. Als Salze kommen Salze einer anorganischen oder organischen Säure in Betracht. Insbesondere werden Salze der Salzsäure oder der Maleinsäure bereitgestellt, so etwa Rosiglitazonmaleat oder Pioglitazonhydrochlorid.

Erfindungsgemäß wird auch eine pharmazeutische Zusammensetzung bereitgestellt, die einen PPARy-Agonisten oder MPC-Inhibitor enthält, und optional einen pharmazeutischen Träger enthält. Sie dient ebenso zur Verwendung in einem Verfahren zur Prävention oder Therapie einer primären, angeborenen Kardiomyopathie bzw. wird zur Prävention oder Therapie einer primären, angeborenen Kardiomyopathie verwendet, insbesondere der vorgenannten primären, angeborenen Kardiomyopathien ausgewählt aus der Gruppe, bestehend aus hypertropher Kardiomyopathie (HCM), dilatativer Kardiomyopathie (DCM), restriktiver HCM, einer Kardiomyopathie bei strukturellen angeborenen Herzfehlern, Erkrankungen aus dem Noonan Syndrom Spektrum oder anderen RASopathien und anderen angeborenen systemischen Erkrankungen, insbesondere hypertropher Kardiomyopathie (HCM).

In funktioneller Hinsicht sind die erfindungsgemäß eingesetzten PPARγ-Agonisten oder MPC-Inhibitoren insbesondere dadurch weiter charakterisiert, daß sie zudem eine Hemmung des Hypoxie-induzierbaren Faktors 1 Alpha bewirken.

Die erfindungsgemäß einzusetzende pharmazeutische Zusammensetzung kann ein racemisches Gemisch eines Glitazons oder ein reines oder angereichertes (*S*)-Enantiomer oder (*R*)-Enantiomer eines Glitazons, bevorzugt mit einem Deuteriumatom am chiralen Zentrum, enthalten. Ein racemisches Gemisch als Wirkstoff der pharmazeutischen Zusammensetzung kann bspw. dann bevorzugt sein, wenn beide Enantiomere mechanistisch wirksam sein sollen. Hingegen werden angereicherte oder Enantiomer-Reinformen dann bevorzugt sein, wenn ein spezifischer, auf dem entsprechenden Enantiomer beruhender Wirkmechanismus bevorzugt ist und/oder der Wirkmechanismus des anderen Enantiomers ausgeschlossen werden soll. So kann ein (*R*)-Enantiomer in der pharmazeutischen Zusammensetzung dann bevorzugt sein, wenn bspw. der PPARγ-Agonismus vermieden und ein biologischer Effekt als MPC-Inhibitor erhöht werden soll. Oder aber das (*S*)-Enantiomer in der Zusammensetzung kann in angereicherter oder in Reinform vorliegen, wenn vorzugsweise ein PPARγ-basierter Wirkmechanismus avisiert ist. "Angereichert" in diesem Kontext bedeutet insbesondere, daß die eine Enantiomer-Fraktion mindestens 70%, vorzugsweise mindestens 80% der Gesamtgewichtsmenge des Wirkstoffs in der pharmazeutischen Zusammensetzung ausmacht.

Die pharmazeutische Zusammensetzung kann in flüssiger, halbflüssiger, gelartiger oder insbesondere fester Form vorliegen. Sie kann, je nach Erfordernis, auf unterschiedlichen Verabreichungswegen verabreicht werden. Bevorzugt ist eine orale Verabreichung, insbesondere in Tablettenform, z.B. als Filmtablette. Ggf. kommt aber auch eine intrakardiale oder intravenöse Verabreichung, insbesondere in Form einer Injektion oder Infusion, in Betracht.

Die pharmazeutische Zusammensetzung gemäß der vorliegenden Erfindung kann optional einen Zusatzstoff enthalten. Beispiele für den Zusatzstoff kann eines oder mehr von Hilfsstoffen, Sprengmitteln, Bindemitteln, Gleitmitteln, Farbstoffen, pH-Einstellmitteln, Tensiden, Stabilisatoren, Korrigentien, Süßstoffen, Aromastoffen, Fluidisatoren, Antistatika, Lichtschutzmitteln, Antioxidantien, Reduktionsmitteln, Chelatbildnern sein. Diese können, bspw. auch zwei oder mehr dieser Zusatzstoffe, in einem geeigneten Verhältnis gemischt und verwendet werden.

Beispiele für einen Hilfsstoff sind kristalline Cellulose, wasserfreies Calciumphosphat, wasserfreies zweibasiges Calciumphosphat, Calciumhydrogenphosphat, gefälltes Calciumcarbonat, Calciumsilikat, Pulvercellulose, Gelatine, leichte wasserfreie Kieselsäure (z. B. leichte wasserfreie Kieselsäure ohne Hydrophobierungsbehandlung oder amorphes Siliciumdioxid-Feinteilchen mit einer Teilchengröße von mehr als 0,1 Mikron), synthetisches Aluminiumsilikat, Magnesiumalumino-Metasilikat, Magnesiumoxid, Calciumphosphat, Calciumcarbonat und Calciumsulfat. Von diesen ist die kristalline Cellulose bevorzugt, z.B. CEOLUS KG801, KG802, PH101, PH102, PH301, PH302, PH-F20, RC-A591 NF (Handelsnamen, hergestellt von Asahi Kasei Chemicals Corporation), einschließlich der sogenannten makrokristallinen Cellulose.

Beispiele für ein Sprengmittel sind Carboxymethylcellulose, Carboxymethylcellulose-Calcium (Carmellose-Calcium), Natrium-Carboxymethylstärke, Carmellose-Natrium, Croscarmellose-Natrium, niedrig-substituierte Hydroxypropylcellulose [vorzugsweise, niedrig-substituierte Hydroxypropylcellulose mit einem Gehalt an Hydroxypropoxygruppen von 5-16 Gew.-%, wie LH-11, LH-21, LH-31, LH-22, LH-32, LH-20, LH-30, LH-33, LH-B1, NBD-020, NBD-021, NBD-022 (Handelsnamen, hergestellt von Shin-Etsu Chemical Co., Ltd.) und dergleichen. Beispiele für ein Bindemittel sind Hydroxypropylcellulose, vorzugsweise, HPC-SSL, SL, L (Handelsnamen, NIPPON SODA CO., LTD.), Hydroxypropylmethylcellulose, Povidon (Polyvinylpyrrolidon), Gummiarabikum-Pulver, Gelatine, Pullulan, Methylcellulose, kristalline Cellulose, niedrig-substituierte Hydroxypropylcellulos, vorzugsweise, niedrig-substituierte Hydroxypropylcellulose mit einem Gehalt an Hydroxypropoxygruppen von 5-16 Gew.-%, wie LH-11, LH-21, LH-31, LH-22, LH-32, LH-20, LH-30, LH-33, LH-B1, NBD-020, NBD-021, NBD-022 (Handelsnamen, hergestellt von Shin-Etsu Chemical Co., Ltd.), Dextran und Polyvinylalkohol.

Beispiele für ein Schmiermittel sind Stearinsäure, Magnesiumstearat, Calciumstearat, Talkum, Saccharoseester von Fettsäuren, Natriumstearylfumarat, Wachse, DL-Leucin, Natriumlaurylsulfat, Magnesiumlaurylsulfat, Macrogol und leichte wasserfreie Kieselsäure (leichte wasserfreie Kieselsäure ohne Hydrophobierungsbehandlung oder amorphes Siliciumdioxid-Feinteilchen mit einer Teilchengröße von mehr als 0,1 Mikron). Von diesen ist Natriumstearylfumarat bevorzugt. Beispiele für einen Farbstoff umfassen Lebensmittelfarben wie Food Color Yellow No. 5 (Sunset Yellow, gleich wie US Food Color Yellow No. 6), Food Color Red No. 2, Food Color Blue No. 2 und dergleichen, Lebensmittellackfarben, gelbes Eisenoxid (gelbes Eisenoxidpigment), rotes Eisenoxid (rotes Eisenoxidpigment), schwarzes Eisenoxid (schwarzes Eisenoxidpigment), Riboflavin, Riboflavin-organischer Säureester (z. B., Riboflavinbuttersäureester), Riboflavinphosphat oder Alkali- oder Erdalkalimetallsalz davon, Phenolphthalein, Titanoxid, Lycopin und Beta-Carotin.

Beispiele für ein pH-Einstellmittel sind Citrat, Phosphat, Carbonat, Tartrat, Fumarat, Acetat und Aminosäuresalz, Ascorbinsäure, (wasserfreie) Zitronensäure, Weinsäure und Apfelsäure. Beispiele für ein Tensid sind Natriumlaurylsulfat, Polysorbat 80, Polyoxyethylen-(160)-Polyoxypropylen-(30)-Glykol, Polyoxyethylen-(196)-Polyoxypropylen-(67)-Glykol und polyoxyethylenhydriertes Rizinusöl 60. Beispiele für einen Stabilisator sind Natriumascorbat, Tocopherol, Tetranatriumedetat, Nicotinsäureamid, Cyclodextrine; Erdalkalimetallsalz (z. B. Calciumcarbonat, Calciumhydroxid, Magnesiumcarbonat, Magnesiumhydroxid, Magnesiumsilikat, Magnesiumaluminat) und Butylhydroxyanisol. Beispiele für einen Süßstoff sind Aspartam, Acesulfam-Kalium, Thaumatin, Saccharin-Natrium und Dikalium-Glycyrrhizinat. Beispiele für einen Aromastoff sind Menthol, Pfefferminzöl, Zitronenöl und Vanillin.

Beispiele für ein Fluidisierungsmittel sind leichte wasserfreie Kieselsäure (leichte wasserfreie Kieselsäure ohne Hydrophobierungsbehandlung oder amorphe Kieselsäure-Feinpartikel mit einer Partikelgröße von mehr als 0,1 Mikron) und hydratisiertes Siliciumdioxid. Hier muss die leichte wasserfreie Kieselsäure nur hydratisiertes Siliziumdioxid (SiO₂ nH₂O) (n ist eine ganze Zahl) als Hauptkomponente enthalten, und konkrete Beispiele dafür sind Sylysia 320 (Handelsname, Fuji Silysia Chemical Ltd.), AEROSIL 200 (Handelsname, NIPPON AEROSIL) und dergleichen. Beispiele für ein Antistatikum sind Talkum und leichte wasserfreie Kieselsäure (leichte wasserfreie Kieselsäure ohne Hydrophobierungsbehandlung oder amorphe Kieselsäure-Feinpartikel mit einer Partikelgröße von mehr als 0,1 Mikron). Beispiele für ein lichtabschirmendes Mittel sind Titanoxid. Beispiele für ein Antioxidans sind Butylhydroxytoluol (BHT), Butylhydroxyanisol (BHA), Tocopherol, Tocopherolester (z. B. Tocopherolacetat), Ascorbinsäure oder deren Alkali- oder Erdalkalimetallsalz, Lycopin und Beta-Carotin. Beispiele für ein Reduktionsmittel sind Cystin und Cystein. Beispiele für einen Chelatbildner sind EDTA oder dessen Alkali- oder Erdalkalimetallsalze.

Eine Formulierung einer pharmazeutischen Zusammensetzung der vorliegenden Erfindung, insbesondere in Form einer Tablette, kann nach üblichen Verfahren und unter Verwendung der oben genannten Zusatzstoffe hergestellt werden. So kann die Formulierung beispielsweise durch Mischen des PPARy-Agonisten oder MPC-Inhibitors oder eines Salzes davon bzw. des Glitazons oder eines Salzes davon (z. B. Pioglitazonhydrochlorid), und eines gegebenenfalls hinzuzufügenden Hilfsstoffs, durch Granulieren der Mischung, Trocknen des Granulats, Sieben desselben, falls erforderlich, Mischen eines gegebenenfalls hinzugefügten Gleitmittels (z.B. Natriumstearylfumarat) damit und Formpressen des erhaltenen Granulats oder der Mischung hergestellt werden. Das Mischen kann mit einer Mischmaschine, wie z. B. einem V-Mischer, einem Taumelmischer und dergleichen, durchgeführt werden. Die Granulierung kann z. B. mit einem Hochgeschwindigkeits-Mischgranulator, einem Wirbelschichttrockner-Granulator und dergleichen erfolgen. Eine Komprimierung kann erfolgen.

Die Formgebung der Tablette kann durch Stanzen erfolgen, z.B. mit einer Einzelstempel-Tablettiermaschine, einer Rundtakt-Tablettiermaschine und dergleichen.

Erforderlichenfalls kann ein Überzug nach einem im technischen Bereich von pharmazeutischen Zubereitungen üblichen Verfahren aufgebracht werden, insbesondere im Falle einer Formulierung als Retard-Präparat. Zusätzlich können Markierungen oder Buchstaben zur Kennzeichnung oder auch eine Trennlinie zur Unterteilung der Tablette aufgebracht werden. Beispiele für die Überzugsbasis sind Zuckerüberzugsbasis, wasserlösliche Filmüberzugsbasis, magensaftresistente Filmüberzugsbasis, Filmüberzugsbasis mit verzögerter Freisetzung und dergleichen.

Als Zuckerüberzugsbasis kann Saccharose verwendet werden, und ferner können eine oder mehrere Arten, ausgewählt aus Talk, gefälltem Calciumcarbonat, Gelatine, Gummi arabicum, Pullulan, Carnaubawachs und dergleichen, in Kombination verwendet werden. Beispiele für die wasserlösliche Filmbeschichtungsbasis umfassen Cellulosepolymere wie Hydroxypropylcellulose, Hydroxypropylmethylcellulose, Hydroxyethylcellulose, Methylhydroxyethylcellulose und dergleichen; synthetische Polymere, wie Polyvinylalkohol, Polyvinylacetaldiethylaminoacetat, Aminoalkylmethacrylat-Copolymer E [Eudragit E (Handelsname)], Polyvinylpyrrolidon und dergleichen; Polysaccharide, wie Pullulan und dergleichen. Beispiele für die magensaftresistente Filmbeschichtungsbasis umfassen Cellulosepolymere wie Hydroxypropylmethylcellulosephthalat, Hydroxypropylmethylcelluloseacetatesuccinat, Carboxymethylethylcellulose, Celluloseacetatphthalat und dergleichen; Acrylsäurepolymere, wie Methacrylsäure-Copolymer L [Eudragit L (Handelsname)], Methacrylsäure-Copolymer LD [Eudragit L-30D55 (Handelsname)], Methacrylsäure-Copolymer S [Eudragit S (Handelsname)] und dergleichen; natürlich vorkommende Substanzen wie Schellack.

Beispiele für die Filmbeschichtungsbasis mit verzögerter Freisetzung umfassen Cellulosepolymere wie Ethylcellulose, Acetylcellulose und dergleichen; Acrylsäurepolymere wie Aminoalkylmethacrylat-Copolymer RS [Eudragit RS (Handelsname)], Ethylacrylat-Methylmethacrylat-Copolymersuspension [Eudragit NE (Handelsname)] und dergleichen.

Zusätzlich kann ein Beschichtungsadditiv zur Beschichtung verwendet werden. Beispiele für den Beschichtungszusatz sind Lichtschutzmittel und/oder Farbstoffe wie Titanoxid, Talkum, rotes Eisenoxid und dergleichen; Weichmacher wie Polyethylenglykol, Triethylcitrat, Rizinusöl, Polysorbate und dergleichen.

Die pharmazeutische Zusammensetzung, enthaltend den PPARy-Agonisten oder den MPC-Inhibitor nach der vorliegenden Erfindung, kann in einer Ausführungsform zur sofortigen Freisetzung oder in einer anderen Ausführungsform zur verzögerten Freisetzung formuliert sein.

Im Falle einer Ausführungsform als Retard-Formulierung mit verzögerter Freisetzung des Wirkstoffs weist eine solche Retard-Formulierung typischerweise eine Freisetzung von 25-58% nach zwei Stunden und 60-100% nach 4h auf. Die Freisetzung kann nach dem Paddle-Verfahren (US Pharmacopeia) in einem Auflösungstestsystem mit 50 rpm (KCL/HCl-Puffer (pH=2) bei 37°C als Testlösung) bestimmt werden. Der Cₘₐₓ-Wert, insbesondere einer Tablette nach oraler Gabe, einer Retard-Formulierung mit 1 mg Wirkstoff ist vorzugsweise innerhalb von 10-90%, bevorzugt zwischen 30 und 80% verglichen mit einer Sofortfreisetzungsformulierung. Vorzugsweise liegt der Cₘₐₓ-Wert also unterhalb der Spitzenkonzentration einer Formulierung mit sofortiger Freisetzung. Unter diesen Bedingungen ist auch der AUC-Wert innerhalb eines Bereichs von 50 bis 150% oder 60 bis 140% verglichen mit einer Sofortfreisetzungsformulierung angestrebt.

Die Formulierung kann als Retard-Präparat dadurch bereitgestellt werden, daß ein Film-überzug mit einer Filmbeschichtungsbasis mit verzögerter Freisetzung, wie oben beschrieben, eingesetzt wird. Alternativ kann die Retard-Formulierung ein Gel-bildendes Polymer, insbesondere ausgewählt aus Polyethylenoxid (z.B. mit einem Molekulargewicht von 100,000 -10,000,000, vorzugsweise 300,000 - 8,000,000), Hypromellose (z.B. mit einem Molekulargewicht von 20,000 - 500,000, vorzugsweise 20,000 - 250,000), Hydroxypropylcellulose, Methylcellulose, Carboxymethylcellulose, Na-Croscarmellose und Mischungen der vorgenannten Polymere enthalten. Der Anteil der Polymerkomponente in der Retard-Formulierung kann zwischen 10 und 90%, vorzugsweise zwischen 30% bis 80% oder 50% bis 80% betragen. Die Freisetzungsrate der Zubereitung mit verzögerter Freisetzung kann durch Veränderung der Menge und des Molekulargewichts des gelbildenden Polymers, wie Polyethylenoxid und dergleichen, gesteuert werden.

Die Retard-Formulierung kann weiterhin bspw. auch einen gelbildenden Promotor enthalten, der das Eindringen von Wasser unterstützt, bevor das gelbildende Polymer zu quellen beginnt. Es kann sich bspw. um eine wasserlösliche hydrophile Verbindung handeln, wie bspw. Lactose, Glucose, Mannitol, Trehalose, D-Sorbitol, Xylitol, Sucrose, Maltose, Lactulose, D-Fructose, Dextran, Glucose oder um wasserlösliche Polymere, wie etwa Polyethyleneglykol. Oder es kann eine wasserunlösliche hydrophile Verbindung eingesetzt werden, z.B. Stärke, Hydroxypropyl-Stärke, Crospovidon, kristalline Cellulose etc.

Im Falle der Herstellung einer derartigen Retard-Formulierung mit einen gelbildenden Polymer kann zur Bereitstellung einer oralen Formulierung, insbesondere einer Tablette, der erfindungsgemäßen pharmazeutischen Zusammensetzung ein gelbildendes Polymer (z.B. Polyethylenoxid) und ggf. ein gelbildender Promotor (z. B. D-Mannitol, Lactose) der zu granulierende Mischung, wie oben beschrieben, hinzugegeben werden. Das Präparat mit verzögerter Freisetzung, enthaltend ein gelbildendes Polymer, kann weiterhin eine schnelle Gelierung erreichen, da seine gelbildende Funktion durch Verkapselung einer Komponente verbessert wird, die das Eindringen von Wasser in das Granulat verhindert (Hydroxypolymer, Bindemittel und dergleichen), und der Weg der Wasserpenetration wird gesichert, indem die Komponente, die das Eindringen von Wasser verhindert, mit einem Oberflächenmodifikator bedeckt wird. Dadurch wird eine Wirkstofffreisetzung nullter Ordnung möglich. Darüber hinaus kann die Festigkeit vermittelt werden, die notwendig ist, um den physikalischen Reizen im Körper durch die Nahrungsaufnahme standzuhalten.

Das Gewicht einer Tablette zur oralen Verabreichung beträgt typischerweise 40 - 600 mg, vorzugsweise 60 - 480 mg, noch bevorzugter 60 - 200 mg, noch bevorzugter 100 - 200 mg.

Der erfindungsgemäß eingesetzte PPARy-Agonist oder der MPC-Inhibitor, insbesondere das erfindungsgemäß eingesetzte Glitazon kann an Kinder oder Erwachsene verabreicht werden. Insbesondere wird erfindungsgemäß eine pädiatrische Therapie, also bspw. von Kindern im Alter von 1 bis 12 Jahren, ermöglicht.

Der PPARγ-Agonist oder der MPC-Inhibitor, insbesondere ein Glitazon als Wirkstoff einer pharmazeutischen Zusammensetzung kann typischerweise in einer Tagesdosierung von 1 bis 30 mg, insbesondere 5 bis 15 mg, je nach Geschlecht, Alter, Körpergewicht, Form der primären, angeborenen Kardiomyopathie, des Verlaufs, der Behandlungsdauer und des Schweregrads und nach Wahl des PPARγ-Agonisten oder des MPC-Inhibitors, insbesondere des Glitazons verabreicht werden. Die Verabreichung kann als Einmal- oder Mehrfachgabe pro Tag erfolgen. Zur Behandlung von Kindern kann eine Formulierung des Wirkstoffes notwendig sein, welche von der Tablettenform abweicht. Bevorzugt können zur pädiatrischen Behandlung Flüssigformulierungen oder Kapselformulierungen sein.

Die erfindungsgemäß eingesetzten PPARy-Agonisten oder MPC-Inhibitoren, insbesondere die erfindungsgemäß eingesetzten Glitazone bzw. eine pharmazeutische Zusammensetzung, enthaltend dieselben, können auch in einer Kombinationstherapie verabreicht werden. So können bspw. zwei oder mehr Wirkstoffe der Wirkstoffklasse der Glitazone miteinander kombiniert werden, also bspw. Rosiglitazon und Pioglitazon. Als weitere Kombinationstherapien kommen ein oder mehr erfindungsgemäß eingesetzte PPARy-Agonist(en) oder MPC-Inhibitor(en), insbesondere ein oder mehr Glitazon/e bzw. eine oder mehr pharmazeutische Zusammensetzung(en), enthaltend dieselben, in Kombination mit mindestens einer Standardtherapie nach dem Stand der Technik in Betracht, welche zur symptomatischen Behandlung eingesetzt werden. Bereitgestellt werden kann diese in Kombination eingesetzte Standardtherapie mittels Gabe eines Wirkstoffs aus der Gruppe ausgewählt aus einem Beta-Blocker, einem Angiotensin Converting Enzyme oder Angiotensin-Umwandlungs-Enzym (ACE-) Hemmer, einem Angiotensin I Rezeptor Blocker, einem Aldosteron-Antagonisten, einem Calcium-Antagonisten, einem Anti-Arrhythmikum und einem Myosin-Modulator, insbesondere zur Reduzierung der Myosin-Aktin-Querbrückenverbindungen, bevorzugt Mavacamten (6-[[(1*S*)-1-phenylethyl]amino]-3-propan-2-yl-1*H*-pyrimidine-2,4-dione).

Offenbart werden auch die Verwendung eines oder mehr der erfindungsgemäß offenbarten PPARy-Agonisten oder MPC-Inhibitoren, insbesondere einer Verbindung aus der Gruppe der Glitazone, zur Herstellung eines Arzneimittels, bspw. einer pharmazeutischen Zusammensetzung wie vorliegend erfindungsgemäß offenbart, zur Prävention oder Behandlung der vorliegend offenbarten Erkrankungen aus der Gruppe der primären, angeborenen Kardiomyopathien.

Schließlich werden erfindungsgemäß Verfahren zur Prävention oder Behandlung von Erkrankungen aus der Gruppe der primären, angeborenen Kardiomyopathien offenbart, wobei einem Subjekt eine pharmazeutisch wirksame Menge eines oder mehrerer PPARγ-Agonisten oder MPC-Inhibitoren, insbesondere eine wirksame Menge eines oder mehrerer Thiazolidin-2,4-dions/e (Glitazons/e) bzw. einer pharmazeutischen Zusammensetzung, enthaltend dieselben, wie vorliegend erfindungsgemäß offenbart, verabreicht wird.

### Abbildungen

Die Abbildungen stellen dar:
Abbildung 1 Pathogenese der hypertrophen Kardiomyopathie

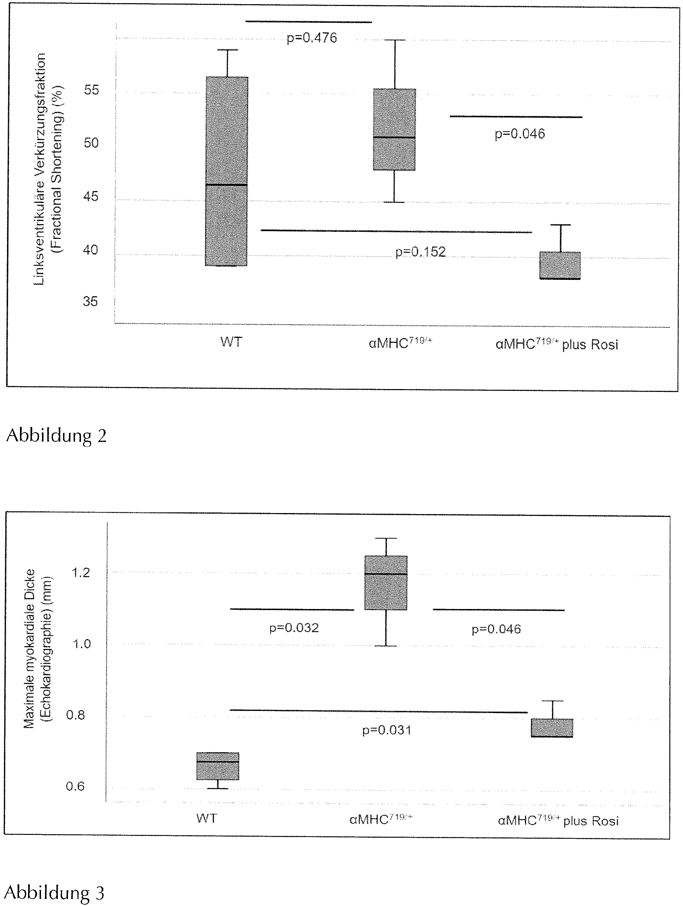

Abbildung 2 Bestimmung der linksventrikulären Verkürzungsfraktion ([(linksventrikulärer enddiastolischer Durchmesser minus linksventrikulärer endsystolischer Durchmesser) dividiert durch linksventrikulärer enddiastolischer Durchmesser] x 100) in Prozent mittels Echokardiographie als Korrelat für die myokardiale Hyperkontraktilität: Die in unbehandelten HCM-Mäusen (αMHC^{719/+}) 6 Wochen nach Versuchsbeginn beobachtete Entwicklung einer hyperdynamen linksventrikulären Kontraktilität gegenüber Wildtyp (WT) Mäusen (p=nicht signifikant) wird durch orale Behandlung mit Rosiglitazon (Rosi) signifikant inhibiert (p=0.046). Es zeigt sich bei den mit Rosiglitazon (5 mg/kg Körpergewicht/Tag oral) behandelten αMHC^{719/+}-Mäusen eine verringerte linksventrikuläre Verkürzungsfraktion im Vergleich zu unbehandelten αMHC^{719/+}-Mäusen.
Abbildung 3 Bestimmung der maximalen Dicke der Wand des linken Herzventrikels mittels Echokardiographie als Korrelat für die myokardiale Hypertrophie: Die in unbehandelten HCM-Mäusen (αMHC^{719/+}) 6 Wochen nach Versuchsbeginn beobachtete Entwicklung einer starken myokardialen Hypertrophie gegenüber Wildtyp (WT) Mäusen (p=0.032) wird durch orale Behandlung mit Rosiglitazon (Rosi) signifikant inhibiert (p=0.046). Es zeigt sich bei den mit Rosiglitazon (5 mg/kg Körpergewicht/Tag oral) behandelten αMHC^{719/+}-Mäusen eine verringerte linksventrikuläre Dicke im Vergleich zu unbehandelten αMHC^{719/+}-Mäusen.

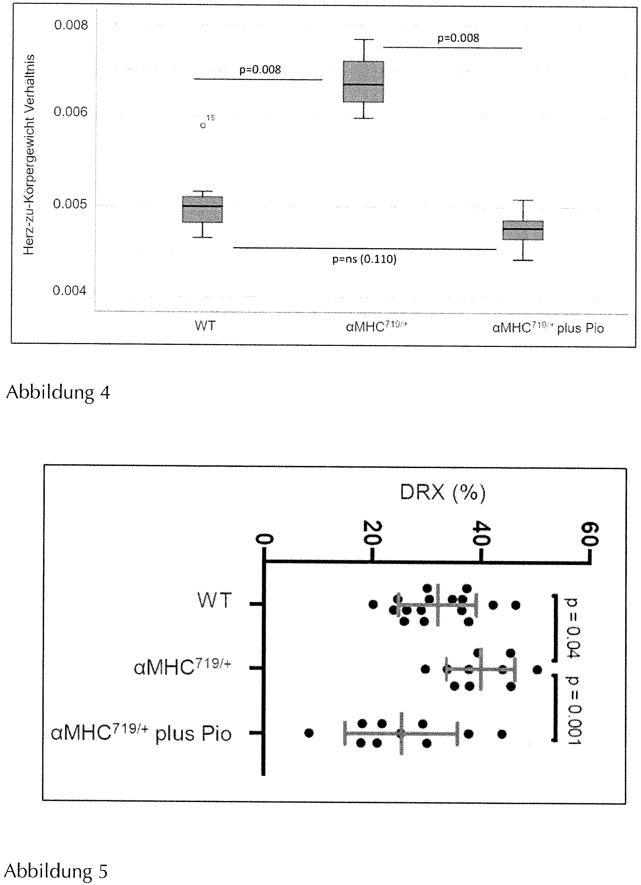

Abbildung 4: Darstellung des Herz-zu-Körpergewicht-Verhältnisses (relative Herzmuskelmasse *ex vivo* nach Organentnahme) als Korrelat für die myokardiale Hypertrophie: Die in unbehandelten HCM-Mäusen (αMHC^{719/+}) 6 Wochen nach Versuchsbeginn beobachtete Entwicklung einer starken myokardialen Hypertrophie (p=0.008) gegenüber Wildtyp-Mäusen (WT) wird durch Behandlung mit Pioglitazon (Pio) komplett inhibiert (p=0.008). *Ex vivo* ist das Herz-zu-Körpergewicht Verhältnis bei mit Pioglitazon (10 mg/kg Körpergewicht/Tag oral) behandelten αMHC^{719/+}-Mäusen im Vergleich zu unbehandelten αMHC^{719/+}-Mäusen signifikant verringert.
Abbildung 5 Untersuchung der Anteile der linksventrikulären kardialen Myosinen im DRX (disordered relaxation) gegenüber dem SRX (super-realaxed) -Zustand unter Verwendung eines fluoreszierenden ATP-Analogons. Im Gegensatz zu unbehandelten αMHC^{719/+}-Mäusen führt die Behandlung mit Pioglitazon zu einer signifikanten Verschiebung zum SRX-Zustand, ähnlich den Wildtyp-Mäusen. Die Behandlung des HCM-Mausmodells mit Pioglitazon wirkt sich also auch positiv auf die funktionellen Eigenschaften des linksventrikulären Herzmuskelgewebes aus. Die Ergebnisse dieses Mant-ATP Assays indizieren, dass die Veränderungen der biophysikalischen Eigenschaften der Myosine, welche als ursächlich für das Krankheitsbild der hypertrophen Kardiomyopathie beschrieben sind, durch die Behandlung mit Pioglitazon im Mausmodell verhindert werden konnten. Während die unbehandelten αMHC^{719/+}-Mäuse Merkmale der typischen biophysikalischen Veränderungen aufzeigten (signifikante Erhöhung von Myosinen im "disordered relaxation" (DRX) Zustandes), bewirkte die Behandlung mit Pioglitazon eine Normalisierung dieser Abnormalitäten.

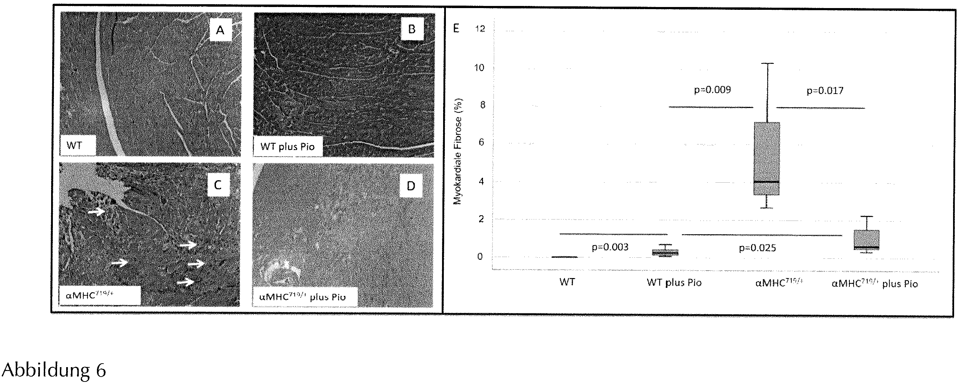

Abbildung 6 Histopathologie des linksventrikulären Myokards von Wildtyp- (WT) und HCM-Mäusen (αMHC^{719/+}) mit und ohne Behandlung mit Pioglitazon (PIO) nach 6 Wochen; in den Masson-Trichrom-gefärbten histologischen Schnitten stellt sich die myokardiale Fibrose in mittelgrauer Farbe dar. Wildtyp-Mäuse weisen kaum Fibrose auf (WT ohne (A) und WT mit (B) Behandlung mit Pioglitazon). Bei HCM-Mäusen findet sich massive myokardiale Fibrose (weiße Pfeile in (C)). Die Behandlung mit Pioglitazon verhindert die Ausbildung myokardialer Fibrose nahezu vollständig (D). (E) Nach semiautomatisierter "threshold detection" von Kollagen in Masson-Trichrom-gefärbten Schnitten im ImageJ Tool zeigt sich signifikant weniger Fibrose in den mit Pioglitazon behandelten HCM-Mäusen im Vergleich zu den unbehandelten HCM-Mäusen (p=0.017). Insgesamt also zeigt sich in der histopathologischen Untersuchung im linksventrikulären Myokard von mit Pioglitazon (10 mg/kg Körpergewicht/Tag oral) behandelten αMHC^{719/+}-Mäusen signifikant weniger Fibrose im Vergleich zu unbehandelten αMHC^{719/+}-Mäusen.

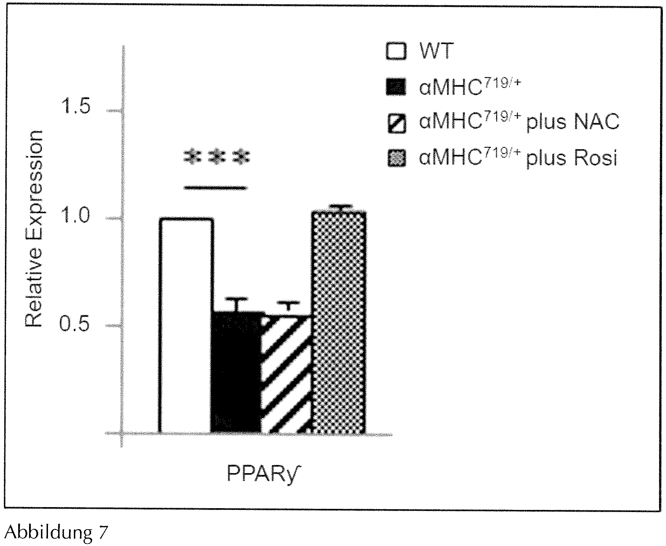

Abbildung 7: Relative Expression der PPARγ-mRNA im linksventrikulären Myokard von unbehandelten und mit N-Azetylcystein (NAC) oder Rosiglitazon (Rosi) behandelten HCM-Mäusen (αMHC^{719/+}) im Vergleich zu Wildtyp-Mäusen (WT) nach 6 Wochen; ***: p<0.001. In Hinblick auf die Wirkung der PPARγ-Agonisten auf die PPARy-mRNA-Expression im Herzmuskel ist festzustellen, dass - verglichen mit Wildtyp Mäusen - sich im Myokard der αMHC^{719/+}-Maus eine signifikant erniedrigte mRNA-Expression von *PPARγ* zeigt. Die orale Gabe von Thiazolidindionen (Rosiglitazon) erhöht die myokardiale mRNA-Expression von *PPARγ* wieder nahezu auf Wildtyp Niveau.

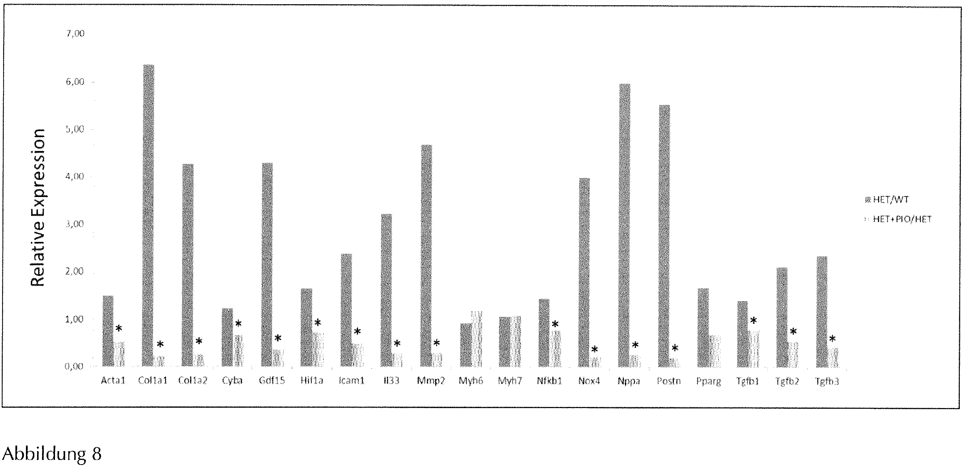

Abbildung 8 Die mRNA-Expression pro-hypertropher, pro-fibrotischer und pro-inflammatorischer Gene wie Acta1, Col1a1,Col1a2, Cyba, Gdf15, Hif1a, Icam1, IL33, Mmp2, Nfkb1, Nox4, Nppa, Postn, Tgfb1, Tgfb2 and Tgfb3 ist im linksventrikulären Myokard von αMHC^{719/+} HCM-Mäusen (HET) im Vergleich zu Wildtyp-Mäusen (WT) erhöht (HET/WT). Durch die Behandlung der HCM-Mäuse mit Pioglitazon (PIO) wird die Überexpression dieser Proteine, die an pro-hypertrophen, pro-fibrotischen und proinflammatorischen Signalwegen beteiligt sind und deren Aktivierung im pathologischen Umbau des Herzmuskels beschrieben ist, signifikant vermindert bzw. komplett verhindert (HET+PIO/HET). *: p<0.001.

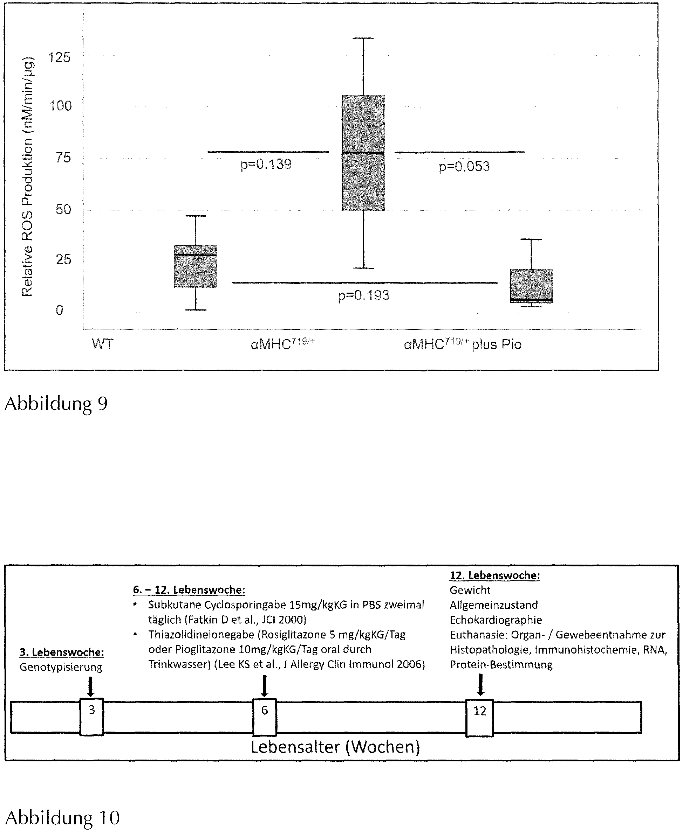

Abbildung 9 Die Bildung freier Sauerstoffradikale (Radical Oxygen Species; ROS) als Ausdruck von oxidativem Stress, ermittelt durch Elektronenspinresonanzspektroskopie (Electron Paramagnetic Resonance Spectroscopy; EPR), zeigt sich bei HCM-Mäusen (αMHC^{719/+}) gegenüber Wildtyp-Mäusen (WT) im linksventrikulären Myokard 6 Wochen nach Versuchsbeginn deutlich erhöht. Die Produktion freier Sauerstoffradikale im linksventrikulären Myokard von mit Pioglitazon behandelten HCM-Mäusen ist gegenüber unbehandelten HCM-Mäusen deutlich verringert (p=0.053) und vergleichbar mit den bei Wildtyp-Mäusen gemessenen Werten. Reduktion des oxidativen Stresses: Die Behandlung mit PPARy-Agonisten führt zu einer verminderten Expression des Hypoxie-induzierbaren Faktors 1 Alpha, einem wesentlichen Transkriptionsfaktor im Redox-Signalweg. Hierdurch werden weniger freie Sauerstoffradikale im linksventrikulären Myokard produziert, wodurch zellulärer oxidativer Stress reduziert wird. Eine protektive Wirkung auf den pathologischen Umbau des Herzmuskels wurde beobachtet.
Abbildung 10 Versuchsplanung/-protokoll

### Ausführungsbeispiele

### Präklinische Studien zur Behandlung der HCM mit Thiazolidindionen

### Versuchsaufbau

Es wurden Wildtyp-Mäuse und genetisch veränderte Mäuse behandelt und mit unbehandelten Mäusen verglichen. Die Untersuchung gentechnisch veränderter Mausstämme erlaubt die Beurteilung der Relevanz eines bestimmten Gens für die pathophysiologischen Phänomene. Bei der durch Sarkomer-Mutationen versursachten hypertrophen Kardiomyopathie kommt es im Herzen der betroffenen Patienten zu progressiver myokardialer Hypertrophie, gestörter diastolischer und systolischer Ventrikelfunktion, strukturellem Umbau des Myokards mit chaotischer Anordnung der Myozyten ("myocyte disarray") sowie Fibrose und zu Rhythmusstörungen. Da der Phänotyp bestimmter genetisch generierter Mausmodelle für die hypertrophe Kardiomyopathie dem Phänotypen der betroffenen Patienten sehr ähnlich ist, eignen sich diese Modelle zur ganzheitlichen Untersuchung der Erkrankung ^{36,37}.

Die Untersuchungen wurden an einem etablierten HCM-Mausmodell durchgeführt, in welchem durch homologe Rekombination eine Arginin-zu-Tryptophan-Veränderung an der Position 719 der schweren Kette des kardialen Maus-alpha-Myosin-Gens bewirkt wurde (bezeichnet als αMHC^{719/+}) ³⁸. Derart genetisch veränderte Mäuse entwickeln mit fortschreitendem Lebensalter zunehmend die klassischen pathognomonischen Krankheitsmerkmale der hypertrophen Kardiomyopathie wie myokardiale Hypertrophie und Fibrose. Der Phänotyp dieses genetisch generierten HCM-Mausmodells simuliert somit den Phänotyp betroffener HCM-Patienten ³⁶, ³⁷, ³⁸. Die Ausprägung der Erkrankung kann durch subkutane Gabe des Calcineurin-Inhibitors Cyclosporin A (CsA) akzeleriert werden ⁹.

Die Genotypisierung der Tiere erfolgte in der 3. Lebenswoche. Zwischen der 6. und der 12. Lebenswoche erfolgte die subkutane Injektion von Cyclosporin A (CsA) aller Mäuse zur Akzelerierung des hypertrophen Remodelings im Myokard im HCM-Mausmodell ⁹. Die eine Hälfte der Mäuse wurde für 6 Wochen zwischen der 6. und 12. Lebenswoche mit den PPARy-Agonisten Rosiglitazon und Pioglitazon aus der Gruppe der Thiazolidindionen behandelt. In der 12. Lebenswoche erfolgte die transthorakale Echokardiographie *in vivo.* Danach wurden die Tiere zur Durchführung der *ex vivo* Untersuchungen (Organentnahme, Histopathologie, und Bestimmung freier Sauerstoffradikale, der mRNA- und der Protein-Expression im Gewebe) euthanisiert (siehe Abbildung 10).D

Für die Versuche wurden ausschließlich männliche Mäuse verwendet, da die männlichen Tiere der hypertrophen Kardiomyopathie Mauslinie (αMHC^{719/+}) im Vergleich zu den weiblichen Tieren einen ausgeprägteren Krankheitsphänotyp aufweisen ³⁹, ³⁷, ³⁸.
Die Gruppengröße betrug je 11 Tiere, zur Analyse wurden pro Gruppe mindestens 6 Tiere miteinander verglichen.

Die *in vivo* Phänotypisierung der linksventrikulären Wanddicke und der Ventrikelfunktion erfolgte mittels transthorakaler Echokardiographie, wie zuvor beschrieben ⁴⁰, ⁴¹, ³⁹.

Konkret erfolgt die echokardiographische Untersuchung wie folgt: Entnahme der Mäuse aus dem Käfig, Wiegen und inspektorische Beurteilung der Tiere, nach 30-minütigem Beobachtungszeitraum leichte Sedierung mit intraperitonealem Midazolam, danach Durchführung der Echokardiographie nach standardisierten publizierten Standardprotokollen ³⁹, ⁴¹, ³⁷, ⁴⁰.

Zur Berechnung der Herzgewicht/Körpergewicht Ratio wurden die Mäuse gewogen und euthanasiert. Der Thorax wurde rasch geöffnet, das Herz entnommen und zweimal in kalter PBS Lösung gewaschen. Die großen Gefäße und das Bindegewebe wurden entfernt, das Herz trockengetupft, gewogen, und das Herz- und Körpergewicht der Maus bestimmt.

Zur Quantifikation myokardialer Fibrose wurden die entnommenen Herzen in 4% Paraformaldehyd fixiert, entwässert, und in Paraffin eingelegt, wie zuvor beschrieben ^{37, 42, 43}. 5-µm Schnitte wurden mit Masson Trichrom und Hematoxylin-Eosin gefärbt. Der Anteil der myokardialen Fibrosierung wurde im digitalisierten Verfahren quantifiziert, indem der Prozentsatz der in Masson Trichrom in blau gefärbten Anteile des Herzmuskels in Relation zum gesamt gefärbten Herzmuskel errechnet wurde. Perivaskuläre und intramurale Strukturen, das Endokard, oder Trabekel wurden bei der Berechnung des fibrotischen Anteiles ausgeschlossen.

Die Messung der Aktivität kardialer Myosin ATPase erfolgte mittels Mant-ATP Assay. Hierbei wird die Kardiomyozytenkontraktilität und -relaxation indirekt mittels Messung der Aktivität kardialer Myosin ATPase untersucht. Hierzu wurde der Mant-ATP-Assay (Roche) am in flüssigem Stickstoff blitzgefrorenen und bei -80°C tiefgefroren aufbewahrten linksventrikulären Myokard der verschiedenen Mausgruppen angewendet. Mant-ATP ist ein fluoreszierendes, nicht-hydrolysables ATP, welches den Kopf des kardialen Myosins bindet. Der Assay misst die Ausschüttung von Mant-ATP nach Zugabe von ATP ⁴⁴, ⁴⁵.

Zur Berechnung der Produktion freier Sauerstoffradikale (ROS) im linksventrikulären Myokard wurde die Elektronenspinresonanz (electron paramagnetic resonance, EPR) angewendet wie beschrieben ⁴⁶⁻⁴⁸. Zusammenfassend wurde linksventrikuläres Myokard zweimal in Krebs-HEPES Buffer gewaschen (pH 7.35; 99 mM NaCl, 4.69 mM KCl, 25 mM NaHCO₃, 1.03 mM KH₂PO₄, 5.6 mM D-Glucose, 20 mM Na-HEPES, 2.5 mM CaCl₂, 1.2 mM MgSO₄) und anschließend in Krebs-HEPES Buffer, welcher mit 25 µM Desferoxamine, 5 µM Diethyldithiocarbamate und 100 µM des Spintrap 1-hydroxy-3-methoxycarbonyl-2 ,2,5,5-tetramethylpyrrolidine (CMH; Noxygen) versehen ist, für 20 Minuten auf Eis inkubiert. Die Analyse der ROS Generation erfolgte bei 37 °C für 10 Minuten mit 20 Scans in einem Escan EPR Spectrometer unter Temperaturkontrolle (Noxygen) und folgenden Parametern: microwave power 23.89 mW; center field 3459-3466 G; steep width 10 G, frequency 9.7690 GHz und modulation amplitude 2.93 G. ROS Generation wurde mittels linearer Regression errechnet und zu dem zellulären Proteingehalt normalisiert.

Die statistische Analyse erfolgte mittels SPSS (Statistical Package for Social Sciences, IBM, Chicago, IL, Version 22). Die Daten werden in Boxplots als Median, Quartile, minimale und maximale Werte gezeigt. Mediane der unterschiedlichen Gruppen wurden mit dem Mann-Whitney-Test (Wilcoxon-Test, nicht-parametrische Daten) geprüft.

Die RNA Isolierung erfolgte mittels peqGOLD Total RNA Kit (Peqlab) nach Angaben des Herstellers aus dem linksventrikulären Myokard. First-strand cDNA wurde aus 500ng total RNA synthetisiert, welche mit random hexamers (TIB Molbiol) reverse-transcribiert wird (Reverse Transcriptase Kit, Invitrogen). RtPCR Analyse wird mit dem Perfecta SYBR Green fast Mix (VWR, Germany) im QIAGEN Rotor-Gene Q (Corbett Rotor-Gene 6000) durchgeführt. Die Quantifikation erfolgt mittels ΔCT Berechnung und Randomisierungstest ^{49, 50}.

Die strangspezifische, polyA-angereicherte RNA-Sequenzierung erfolgte wie zuvor beschrieben ⁵¹. Die Isolierung der RNA erfolgte mit dem AllPrep RNA Kit (Qiagen), die Bestimmung der RNA-Integritätszahl (RIN) mit dem Agilent 2100 BioAnalyzer (RNA 6000 Nano Kit, Agilent). Für die Präparation der Bibliothek wurde 1 µg RNA poly(A)-selektiert, fragmentiert und mit dem Elute, Prime, Fragment Mix (Illumina) reverse-transcribiert. A-tailing, Adaptor-Ligation und Bibliotheksanreicherung wurden wie in der TruSeq Stranded mRNA Sample Prep Guide (Illumina) beschrieben durchgeführt. RNA-Bibliotheken wurden mit dem Agilent 2100 BioAnalyzer und dem Quant-iT PicoGreen dsDNA Assay Kit (Life Technologies) auf Qualität und Quantität geprüft. Die RNA-Bibliotheken wurden als 100 bp Paired-End-Läufe auf einer Illumina HiSeq4000-Plattform sequenziert. Der STAR Aligner (Version 2.4.2a) ⁵² mit modifizierten Parametereinstellungen (--twopassMode=Basic) wurde für das Split-Read-Alignment gegen die humane Genomassembly hg19 (GRCh37) und die UCSC knownGene-Annotation verwendet. Zur Quantifizierung der Anzahl der Reads, die auf annotierte Gene abgebildet werden, wurde HTseq-count (Version 0.6.0) ⁵³ verwendet. FPKM-Werte (Fragments Per Kilobase of transcript per Million fragments mapped) wurden mithilfe von benutzerdefinierten Skripten berechnet. Die Analyse der differentiellen Expression wurde mit dem R Bioconductor-Paket DESeq2 ⁵⁴ durchgeführt.

Zusammenfassend weisen die Ergebnisse im präklinischen αMHC^{719/+} HCM-Mausmodell - in Analogie zur häufigsten genetischen Mutation bei HCM-Patienten - nach, daß eine Herunterregulierung von PPARγ im linksventrikulären Myokard im αMHC^{719/+}-Mausmodell gegenüber der Wildtyp-Maus erfolgt und dass die orale Gabe der PPARγ-Agonisten Rosiglitazon und Pioglitazon zum einen die Veränderung der biophysikalischen Eigenschaften in der Herzmuskelzelle, und zum anderen die Entstehung von myokardialer Hypertrophie und Fibrose im HCM-Mausmodell weitgehend verhindert. Die echokardiographisch bestimmte Hyperkontraktilität und die starke Zunahme der Dicke des linken Ventrikels in der HCM-Maus gegenüber der Wildtyp-Maus wird durch Behandlung mit dem PPARγ-Agonisten Rosiglitazon signifikant vermindert. Die Behandlung mit Pioglitazon verhindert auch die im linksventrikulären Myokard des HCM-Mausmodells beschriebenen funktionellen biophysikalischen Veränderungen und die Überexpression pro-hypertropher, pro-fibrotischer und pro-inflammatorischer Gene. Zumindest zum Teil werden diese protektiven Effekte vermutlich unter anderem durch die Hemmung der Aktivierung des Hypoxie-induzierbaren-Faktors alpha (HIF1α) ⁵⁵ verursacht. In der Folge kommt es zur Inhibierung des Redox-Systems und zur Reduktion des myokardialen oxidativen Stresses. Diese Hypothese wird auch durch die signifikant erniedrigten Messwerte freier Sauerstoffradikale im linksventrikulären Myokard von mit Pioglitazon behandelten HCM-Mäusen unterstützt. Im präklinischen HCM Mausmodell wurde also gezeigt, dass die medikamentöse Behandlung mit einem PPARγ-Agonisten, wie bspw. dem Thiazolidindion Pioglitazon oder Rosiglitazon, den pathologischen Umbau des Herzmuskels in der hypertrophen Kardiomyopathie signifikant bzw. nahezu vollständig verhindern kann.

### Literatur

1. Maron BJ, Seidman CE, Ackerman MJ, Towbin JA, Maron MS, Ommen SR, Nishimura RA and Gersh BJ. How should hypertrophic cardiomyopathy be classified?: What's in a name? Dilemmas in nomenclature characterizing hypertrophic cardiomyopathy and left ventricular hypertrophy. Circ Cardiovasc Genet. 2009;2:81-5; discussion 86.
2. Maron BJ. Hypertrophic cardiomyopathy: a systematic review. JAMA. 2002;287:1308-20.
3. Maron BJ, McKenna WJ, Danielson GK, Kappenberger LJ, Kuhn HJ, Seidman CE, Shah PM, Spencer WH, Spirito P, Ten Cate FJ, Wigle ED, Vogel RA, Abrams J, Bates ER, Brodie BR, Danias PG, Gregoratos G, Hlatky MA, Hochman JS, Kaul S, Lichtenberg RC, Lindner JR, O'Rourke RA, Pohost GM, Schofield RS, Tracy CM, Winters WL, Klein WW, Priori SG, Alonso-Garcia A, Blomström-Lundqvist C, De Backer G, Deckers J, Flather M, Hradec J, Oto A, Parkhomenko A, Silber S and Torbicki A. American College of Cardiology/European Society of Cardiology Clinical Expert Consensus Document on Hypertrophic Cardiomyopathy. A report of the American College of Cardiology Foundation Task Force on Clinical Expert Consensus Documents and the European Society of Cardiology Committee for Practice Guidelines. 2003;24:1965-1991.
4. Maron BJ, Towbin JA, Thiene G, Antzelevitch C, Corrado D, Arnett D, Moss AJ, Seidman CE, Young JB, American Heart A, Council on Clinical Cardiology HF, Transplantation C, Quality of C, Outcomes R, Functional G, Translational Biology Interdisciplinary Working G, Council on E and Prevention. Contemporary definitions and classification of the cardiomyopathies: an American Heart Association Scientific Statement from the Council on Clinical Cardiology, Heart Failure and Transplantation Committee; Quality of Care and Outcomes Research and Functional Genomics and Translational Biology Interdisciplinary Working Groups; and Council on Epidemiology and Prevention. Circulation. 2006;113:1807-16.
5. Gersh BJ, Maron BJ, Bonow RO, Dearani JA, Fifer MA, Link MS, Naidu SS, Nishimura RA, Ommen SR, Rakowski H, Seidman CE, Towbin JA, Udelson JE and Yancy CW. 2011 ACCF/AHA guideline for the diagnosis and treatment of hypertrophic cardiomyopathy: executive summary: a report of the American College of Cardiology Foundation/American Heart Association Task Force on Practice Guidelines. J Am Coll Cardiol. 58:2703-38.
6. Ommen SR, Mital S, Burke MA, Day SM, Deswal A, Elliott P, Evanovich LL, Hung J, Joglar JA, Kantor P, Kimmelstiel C, Kittleson M, Link MS, Maron MS, Martinez MW, Miyake CY, Schaff HV, Semsarian C and Sorajja P. 2020 AHA/ACC Guideline for the Diagnosis and Treatment of Patients With Hypertrophic Cardiomyopathy: A Report of the American College of Cardiology/American Heart Association Joint Committee on Clinical Practice Guidelines. Circulation. 2020;142:e558-e631.
7. Semsarian C, Ahmad I, Giewat M, Georgakopoulos D, Schmitt JP, McConnell BK, Reiken S, Mende U, Marks AR, Kass DA, Seidman CE and Seidman JG. The L-type calcium channel inhibitor diltiazem prevents cardiomyopathy in a mouse model. J Clin Invest. 2002;109:1013-20.
8. Westermann D, Knollmann BC, Steendijk P, Rutschow S, Riad A, Pauschinger M, Potter JD, Schultheiss HP and Tschope C. Diltiazem treatment prevents diastolic heart failure in mice with familial hypertrophic cardiomyopathy. Eur J Heart Fai/. 2006;8:115-21.
9. Teekakirikul P, Eminaga S, Toka O, Alcalai R, Wang L, Wakimoto H, Nayor M, Konno T, Gorham JM, Wolf CM, Kim JB, Schmitt JP, Molkentin JD, Norris RA, Tager AM, Hoffman SR, Markwald RR, Seidman CE and Seidman JG. Cardiac fibrosis in mice with hypertrophic cardiomyopathy is mediated by non-myocyte proliferation and requires Tgfbeta. J Clin Invest. 120:3520-9.
10. Ho CY, Lakdawala NK, Cirino AL, Lipshultz SE, Sparks E, Abbasi SA, Kwong RY, Antman EM, Semsarian C, Gonzalez A, Lopez B, Diez J, Orav EJ, Colan SD and Seidman CE. Diltiazem treatment for pre-clinical hypertrophic cardiomyopathy sarcomere mutation carriers: a pilot randomized trial to modify disease expression. JACC Heart Fail. 2015;3:180-8.
11. Shimada YJ, Passeri JJ, Baggish AL, O'Callaghan C, Lowry PA, Yannekis G, Abbara S, Ghoshhajra BB, Rothman RD, Ho CY, Januzzi JL, Seidman CE and Fifer MA. Effects of losartan on left ventricular hypertrophy and fibrosis in patients with nonobstructive hypertrophic cardiomyopathy. JACC Heart Fail. 2013;1:480-7.
12. Axelsson A, Iversen K, Vejlstrup N, Ho CY, Havndrup O, Kofoed KF, Norsk J, Jensen M and Bundgaard H. Functional effects of losartan in hypertrophic cardiomyopathy-a randomised clinical trial. Heart. 2016;102:285-91.
13. Kawas RF, Anderson RL, Ingle SRB, Song Y, Sran AS and Rodriguez HM. A smallmolecule modulator of cardiac myosin acts on multiple stages of the myosin chemomechanical cycle. J Biol Chem. 2017;292:16571-16577.
14. Olivotto I, Oreziak A, Barriales-ViIIa R, Abraham TP, Masri A, Garcia-Pavia P, Saberi S, Lakdawala NK, Wheeler MT, Owens A, Kubanek M, Wojakowski W, Jensen MK, Gimeno-Blanes J, Afshar K, Myers J, Hegde SM, Solomon SD, Sehnert AJ, Zhang D, Li W, Bhattacharya M, Edelberg JM, Waldman CB, Lester SJ, Wang A, Ho CY, Jacoby D and investigators E-Hs. Mavacamten for treatment of symptomatic obstructive hypertrophic cardiomyopathy (EXPLORER-HCM): a randomised, double-blind, placebo-controlled, phase 3 trial. Lancet. 2020;396:759-769.
15. Berger J and Moller DE. The mechanisms of action of PPARs. Annu Rev Med. 2002;53:409-35.
16. Kliewer SA, Xu HE, Lambert MH and Willson TM. Peroxisome proliferator-activated receptors: from genes to physiology. Recent Prog Horm Res. 2001;56:239-63.
17. Elbrecht A, Chen Y, Cullinan CA, Hayes N, Leibowitz M, Moller DE and Berger J. Molecular cloning, expression and characterization of human peroxisome proliferator activated receptors gamma 1 and gamma 2. Biochem Biophys Res Commun. 1996;224:431-7.
18. Greene ME, Blumberg B, McBride OW, Yi HF, Kronquist K, Kwan K, Hsieh L, Greene G and Nimer SD. Isolation of the human peroxisome proliferator activated receptor gamma cDNA: expression in hematopoietic cells and chromosomal mapping. Gene Expr. 1995;4:281-99.
19. Zieleniak A, Wojcik M and Wozniak LA. Structure and physiological functions of the human peroxisome proliferator-activated receptor gamma. Arch Immunol Ther Exp (Warsz). 2008;56:331-45.
20. Michalik L, Auwerx J, Berger JP, Chatterjee VK, Glass CK, Gonzalez FJ, Grimaldi PA, Kadowaki T, Lazar MA, O'Rahilly S, Palmer CN, Plutzky J, Reddy JK, Spiegelman BM, Staels B and Wahli W. International Union of Pharmacology. LXI. Peroxisome proliferator-activated receptors. Pharmacological reviews. 2006;58:726-41.
21. Lehrke M and Lazar MA. The many faces of PPARgamma. Cell. 2005;123:993-9.
22. Yu S and Reddy JK. Transcription coactivators for peroxisome proliferator-activated receptors. Biochim Biophys Acta. 2007;1771:936-51.
23. Colca JR, Wyse BM, Sawada G, Jodelis KS, Connell CL, Fletcher-McGruder BL, Palazuk BJ and Diani AR. Ciglitazone, a hypoglycemic agent: early effects on the pancreatic islets of ob/ob mice. Metabolism. 1988;37:276-80.
24. Diani AR, Sawada GA and Wyse BM. A review of the effects of ciglitazone on the pancreatic islets of obese, hyperglycemic mice. Prog Clin Biol Res. 1988;265:193-209.
25. Fujiwara T, Yoshioka S, Yoshioka T, Ushiyama I and Horikoshi H. Characterization of new oral antidiabetic agent CS-045. Studies in KK and ob/ob mice and Zucker fatty rats. Diabetes. 1988;37:1549-58.
26. Sarhangi N, Sharifi F, Hashemian L, Hassani Doabsari M, Heshmatzad K, Rahbaran M, Jamaldini SH, Aghaei Meybodi HR and Hasanzad M. PPARG (Pro12Ala) genetic variant and risk of T2DM: a systematic review and meta-analysis. Sci Rep. 2020;10:12764.
27. Rizos CV, Kei A and Elisaf MS. The current role of thiazolidinediones in diabetes management. Arch Toxicol. 2016;90:1861-81.
28. Karak M, Bal NC, Bal C and Sharon A. Targeting peroxisome proliferator-activated receptor gamma for generation of antidiabetic drug. Current diabetes reviews. 2013;9:275-85.
29. Derosa G and Maffioli P. Peroxisome proliferator-activated receptor-gamma (PPARgamma) agonists on glycemic control, lipid profile and cardiovascular risk. CurrMol Pharmacol. 2012;5:272-81.
30. Spiegelman BM. Peroxisome proliferator-activated receptor gamma: A key regulator of adipogenesis and systemic insulin sensitivity. Eur J Med Res. 1997;2:457-64.
31. Kokeny G, Calvier L, Legchenko E, Chouvarine P, Mozes MM and Hansmann G. PPARgamma is a gatekeeper for extracellular matrix and vascular cell homeostasis: beneficial role in pulmonary hypertension and renal/cardiac/pulmonary fibrosis. Curr Opin Nephrol Hypertens. 2020;29:171-179.
32. Hansmann G, Calvier L, Risbano MG and Chan SY. Activation of the Metabolic Master Regulator PPARgamma: A Potential PIOneering Therapy for Pulmonary Arterial Hypertension. American journal of respiratory cell and molecular biology. 2020;62:143-156.
33. DeWitt S, Czarnik AW and Jacques V. Deuterium-Enabled Chiral Switching (DECS) Yields Chirally Pure Drugs from Chemically Interconverting Racemates. ACS Med Chem Lett. 2020;1 1:1789-1792.
34. Divakaruni AS, Wiley SE, Rogers GW, Andreyev AY, Petrosyan S, Loviscach M, Wall EA, Yadava N, Heuck AP, Ferrick DA, Henry RR, McDonald WG, Colca JR, Simon MI, Ciaraldi TP and Murphy AN. Thiazolidinediones are acute, specific inhibitors of the mitochondrial pyruvate carrier. Proc Natl Acad Sci USA. 2013;110:5422-7.
35. Jamali B, Bjornsdottir I, Nordfang O and Hansen SH. Investigation of racemisation of the enantiomers of glitazone drug compounds at different pH using chiral HPLC and chiral CE. J Pharm Biomed Anal. 2008;46:82-7.
36. Geisterfer-Lowrance AA, Christe M, Conner DA, Ingwall JS, Schoen FJ, Seidman CE and Seidman JG. A mouse model of familial hypertrophic cardiomyopathy. Science. 1996;272:731-4.
37. Wolf CM, Moskowitz IP, Arno S, Branco DM, Semsarian C, Bernstein SA, Peterson M, Maida M, Morley GE, Fishman G, Berul Cl, Seidman CE and Seidman JG. Somatic events modify hypertrophic cardiomyopathy pathology and link hypertrophy to arrhythmia. Proc Natl Acad Sci USA. 2005;102:18123-8.
38. Teekakirikul P, Eminaga S, Toka O, Alcalai R, Wang L, Wakimoto H, Nayor M, Konno T, Gorham JM, Wolf CM, Kim JB, Schmitt JP, Molkentin JD, Norris RA, Tager AM, Hoffman SR, Markwald RR, Seidman CE and Seidman JG. Cardiac fibrosis in mice with hypertrophic cardiomyopathy is mediated by non-myocyte proliferation and requires Tgfbeta. J Clin Invest. 2010;120:3520-9.
39. Fatkin D, McConnell BK, Mudd JO, Semsarian C, Moskowitz IG, Schoen FJ, Giewat M, Seidman CE and Seidman JG. An abnormal Ca(2+) response in mutant sarcomere protein-mediated familial hypertrophic cardiomyopathy. J Clin Invest. 2000;106:1351-9.
40. Wolf CM, Arad M, Ahmad F, Sanbe A, Bernstein SA, Toka O, Konno T, Morley G, Robbins J, Seidman JG, Seidman CE and Berul CI. Reversibility of PRKAG2 glycogen-storage cardiomyopathy and electrophysiological manifestations. Circulation. 2008;117:144-54.
41. Wolf CM, Wang L, Alcalai R, Pizard A, Burgon PG, Ahmad F, Sherwood M, Branco DM, Wakimoto H, Fishman GI, See V, Stewart CL, Conner DA, Berul CI, Seidman CE and Seidman JG. Lamin A/C haploinsufficiency causes dilated cardiomyopathy and apoptosistriggered cardiac conduction system disease. J Mol Cell Cardiol. 2008;44:293-303.
42. Semsarian C, Ahmad I, Giewat M, Georgakopoulos D, Schmitt JP, McConnell BK, Reiken S, Mende U, Marks AR, Kass DA, Seidman CE and Seidman JG. The L-type calcium channel inhibitor diltiazem prevents cardiomyopathy in a mouse model. Journal of Clinical Investigation. 2002; 109:1013-1020.
43. Arad M, Moskowitz IP, Patel VV, Ahmad F, Perez-Atayde AR, Sawyer DB, Walter M, Li GH, Burgon PG, Maguire CT, Stapleton D, Schmitt JP, Guo XX, Pizard A, Kupershmidt S, Roden DM, Berul CI, Seidman CE and Seidman JG. Transgenic mice overexpressing mutant PRKAG2 define the cause of Wolff-Parkinson-White syndrome in glycogen storage cardiomyopathy. Circulation. 2003;107:2850-6.
44. Jahn W. The association of actin and myosin in the presence of gamma-amido-ATP proceeds mainly via a complex with myosin in the closed conformation. Biochemistry. 2007;46:9654-64.
45. LaConte LEW, Srivastava S and Mukherjee K. Probing Protein Kinase-ATP Interactions Using a Fluorescent ATP Analog. Methods Mol Biol. 2017;1647:171-183.
46. Diebold I, Petry A, Hess J and Gorlach A. The NADPH oxidase subunit NOX4 is a new target gene of the hypoxia-inducible factor-1 . Molecular biology of the cell. 2010;21:2087-96.
47. Rzymski T, Petry A, Kracun D, Riess F, Pike L, Harris AL and Gorlach A. The unfolded protein response controls induction and activation of ADAM17/TACE by severe hypoxia and ER stress. Oncogene. 2012;31:3621-34.
48. Hewing B, Ludwig A, Dan C, Potzsch M, Hannemann C, Petry A, Lauer D, Gorlach A, Kaschina E, Muller DN, Baumann G, Stangl V, Stangl K and Wilck N. Immunoproteasome subunit ss5i/LMP7-deficiency in atherosclerosis. Scientific reports. 2017;7:13342.
49. Pfaffl MW. A new mathematical model for relative quantification in real-time RTPCR. Nucleic Acids Research. 2001 ;Vol. 29.
50. Michael W. Pfaffl GHaLD. Relative expression software tool (REST©) for group-wise comparison and statistical analysis of relative expression results in real-time PCR. Nucleic Acids Research. 2002;2002.
51. Haack TB, Kopajtich R, Freisinger P, Wieland T, Rorbach J, Nicholls TJ, Baruffini E, Walther A, Danhauser K, Zimmermann FA, Husain RA, Schum J, Mundy H, Ferrero I, Strom TM, Meitinger T, Taylor RW, Minczuk M, Mayr JA and Prokisch H. ELAC2 mutations cause a mitochondrial RNA processing defect associated with hypertrophic cardiomyopathy. American journal of human genetics. 2013;93:211-23.
52. Dobin A, Davis CA, Schlesinger F, Drenkow J, Zaleski C, Jha S, Batut P, Chaisson M and Gingeras TR. STAR: ultrafast universal RNA-seq aligner. Bioinformatics. 2013;29:15-21.
53. Anders S, Pyl PT and Huber W. HTSeq--a Python framework to work with highthroughput sequencing data. Bioinformatics. 2015;31:166-9.
54. Love MI, Huber W and Anders S. Moderated estimation of fold change and dispersion for RNA-seq data with DESeq2. Genome Biol. 2014;15:550.
55. Lee KS, Kim SR, Park SJ, Park HS, Min KH, Jin SM, Lee MK, Kim UH and Lee YC. Peroxisome proliferator activated receptor-gamma modulates reactive oxygen species generation and activation of nuclear factor-kappaB and hypoxia-inducible factor 1 alpha in allergic airway disease of mice. J Allergy Clin Immunol. 2006;118:120-7.

## Patentansprüche

1. Ein PPARy-Agonist zur Verwendung in einem Verfahren zur Prävention oder Therapie einer primären, angeborenen Kardiomyopathie.

2. PPARγ-Agonist zur Verwendung in einem Verfahren nach Anspruch 1, wobei die primäre, angeborene Kardiomyopathie ausgewählt ist aus der Gruppe, bestehend aus hypertropher Kardiomyopathie (HCM), dilatativer Kardiomyopathie (DCM), restriktiver HCM, einer Kardiomyopathie bei strukturellen angeborenen Herzfehlern und bei RASopathien, insbesondere Erkrankungen aus dem Noonan-Syndrom-Spektrum.

3. PPARγ-Agonist zur Verwendung in einem Verfahren nach Anspruch 1 oder 2, wobei die primäre Kardiomyopathie eine hypertrophe Kardiomyopathie ist.

4. PPARγ-Agonist zur Verwendung in einem Verfahren nach einem der Ansprüche 1 bis 3, wobei der PPARy-Agonist eine Verbindung aus der Gruppe der Thiazolidin-2,4-dione ist.

5. PPARγ-Agonist zur Verwendung in einem Verfahren nach einem der Ansprüche 1 bis 4, wobei der PPARγ-Agonist ein am C5-Ringatom substituiertes Thiazolidin-2,4-dion ist.

6. PPARγ-Agonist zur Verwendung in einem Verfahren nach einem der Ansprüche 1 bis 5, wobei der PPARy-Agonist ein am C5-Ringatom einen Substituenten aufweist, der zwei über eine Linkerstruktur miteinander verbundene (hetero)aromatische Ringsysteme aufweist.

7. PPARγ-Agonist zur Verwendung in einem Verfahren nach einem der Ansprüche 1 bis 6, wobei der PPARγ-Agonist eine Glitazon ist, insbesondere ausgewählt ist aus der Gruppe, bestehend aus Ciglitazon, Balaglitazon, Darglitazon, Englitazon, Netoglitazon, Pioglitazon, Rivoglitazon, Rosiglitazon, GQ-16, und Troglitazon, wobei der PPARγ-Agonist bevorzugt Pioglitazon oder Rosiglitazon ist.

8. PPARγ-Agonist zur Verwendung in einem Verfahren nach einem der Ansprüche 1 bis 7, wobei der PPARγ-Agonist ein Salz einer anorganischen oder organischen Säure, insbesondere ein Salz der Salzsäure oder ein Salz der Maleinsäure, ist.

9. PPARγ-Agonist zur Verwendung in einem Verfahren nach einem der Ansprüche 1 bis 8, wobei der PPARγ-Agonist ein (*S*)-Enantiomer, vorzugsweise ein am chiralen Zentrum deuteriertes (*S*)-Enantiomer, ist.

10. Pharmazeutische Zusammensetzung, enthaltend einen PPARγ-Agonisten nach einem der Ansprüche 1 bis 9 und optional einen pharmazeutischen Träger, zur Verwendung in einem Verfahren zur Prävention oder Therapie einer primären, angeborenen Kardiomyopathie.

11. Pharmazeutische Zusammensetzung zur Verwendung in einem Verfahren nach Anspruch 10, wobei die primäre, angeborene Kardiomyopathien ausgewählt ist aus der Gruppe, bestehend aus hypertropher Kardiomyopathie (HCM), dilatativer Kardiomyopathie (DCM), restriktiver HCM, einer Kardiomyopathie bei strukturellen angeborenen Herzfehlern und RASopathien, insbesondere Erkrankungen aus dem Noonan-Syndrom-Spektrum, oder insbesondere hypertropher Kardiomyopathie (HCM).

12. Pharmazeutische Zusammensetzung zur Verwendung in einem Verfahren nach Anspruch 10 oder 11, wobei die Zusammensetzung ein (*S*)-Enantiomer eines Glitazons, bevorzugt mit einem Deuteriumatom am chiralen Zentrum, in angereicherter oder in Reinform oder ein Racemat des (5)- und des (*R*)-Enantiomers eines Glitazons enthält.

13. Pharmazeutische Zusammensetzung zur Verwendung in einem Verfahren nach einem der Ansprüche 9 bis 12, wobei die pharmazeutische Zusammensetzung eine oral zu verabreichende therapeutische Zusammensetzung, insbesondere eine Filmtablette, ist.

14. PPARγ-Agonist zur Verwendung in einem Verfahren nach einem der Ansprüche 1 bis 9 oder die pharmazeutische Zusammensetzung zur Verwendung in einem Verfahren nach einem der Ansprüche 10 bis 13, wobei der PPARγ-Agonist in einer Tagesdosierung von 1 bis 30 mg, vorzugsweise 1 bis 15 mg verabreicht wird.

15. PPARγ-Agonist zur Verwendung in einem Verfahren nach einem der Ansprüche 1 bis 9 oder die pharmazeutische Zusammensetzung zur Verwendung in einem Verfahren nach einem der Ansprüche 10 bis 13, wobei das Verfahren eine Kombinationstherapie mit einem Wirkstoff ausgewählt aus der Gruppe , bestehend aus einem Beta-Blocker, einem Angiotensin Converting Enzyme oder Angiotensin-Umwandlungs-Enzym (ACE-) Hemmer, einem Angiotensin I Rezeptor Blocker, einem Aldosteron-Antagonisten, einem Calcium-Antagonisten, einem Anti-Arrhythmikum und einem Myosin-Modulator, umfaßt.
